Europäisches Patentamt

European Patent Office

Office européen des brevets

(11) Publication number: **0 158 976 B1**

# EUROPEAN PATENT SPECIFICATION

(45) Date of publication of patent specification: **29.01.92**  (51) Int. Cl.⁵: **C01B 25/45**, B01J 29/04

(21) Application number: **85104386.9**

(22) Date of filing: **11.04.85**

(54) Molecular sieve compositions.

(30) Priority: **13.04.84 US 600166**
**13.04.84 US 599812**
**13.04.84 US 599776**
**13.04.84 US 599771**
**13.04.84 US 599807**
**13.04.84 US 599811**
**13.04.84 US 599809**
**13.04.84 US 599813**
**13.04.84 US 600171**

(43) Date of publication of application:
**23.10.85 Bulletin 85/43**

(45) Publication of the grant of the patent:
**29.01.92 Bulletin 92/05**

(84) Designated Contracting States:
**BE DE FR GB IT NL**

(56) References cited:
**EP-A- 0 043 562**

(73) Proprietor: **UOP**
**25 East Algonquin Road**
**Des Plaines, Illinois 60017-5017(US)**

(72) Inventor: **Flanigen, Edith Marie**
**502 Woodland Hills Road**
**White Plains New York 10603(US)**
Inventor: **Lok, Brent Mei Tak**
**17A Deerwood Drive**
**New City New York 10956(US)**
Inventor: **Patton, Robert Lyle**
**87 Harris Road**
**Katonah New York 10536(US)**
Inventor: **Wilson, Stephen Thomas**
**1024 E. Main Street**
**Shrub Oak New York 10588(US)**

(74) Representative: **Eggert, Hans-Gunther, Dr.**
**Räderscheidtstrasse 1**
**W-5000 Köln 41(DE)**

**Description**

The instant invention relates to a novel class of crystalline microporous molecular sieves, to the method of their preparation and to their use as adsorbents and catalyst. The invention relates to novel molecular sieves having at least three elements, as hereinafter defined, present as framework as tetrahedral oxide units. These compositions may be prepared hydrothermally from gels containing reactive compounds of aluminum and phosphorus and at least one additional element, as hereinafter set forth, capable of forming a framework tetrahedral oxide, and preferably by use of at least one organic templating agent which functions in part to determine the course of the crystallization mechanism and the structure of the crystalline product.

Background of the Invention

Molecular sieves of the crystalline aluminosilicate zeolite type are well known in the art and now comprise over 150 species of both naturally occurring and synthetic compositions. In general the crystalline zeolites are formed from corner-sharing $AlO_2$ and $SiO_2$ tetrahedra and are characterized by having pore openings of uniform dimensions, having a significant ion-exchange capacity and being capable of reversibly desorbing an adsorbed phase which is dispersed throughout the internal voids of the crystal without displacing any atoms which make up the permanent crystal structure.

Other crystalline microporous compositions which are not zeolitic, i.e. do not contain only $AlO_2$ and $SiO_2$ tetrahedra as essential framework constituents, but which exhibit the ion-exchange and/or adsorption characteristics of the zeolites are also known. Metal organosilicates which are said to possess ion-exchange properties, have uniform pores and be capable of reversibly adsorbing molecules having molecular diameters of about 0,6nm (6Å) or less, are reported in U.S. Patent No. 3,941,871 issued March 2, 1976 to Dwyer et al. A pure silica polymorph, silicalite, having molecular sieving properties and a neutral framework containing neither cations nor cation sites is disclosed in U.S. Patent No. 4,061,724 issued December 6, 1977 to R.W. Grose et al.

A recently reported class of microporous compositions and the first framework oxide molecular sieves synthesized without silica, are the crystalline aluminophosphate compositions disclosed in U.S. Patent No. 4,310,440 issued January 12, 1982 to Wilson et al. These materials are formed from $AlO_2$ and $PO_2$ tetrahedra and have electrovalently neutral frameworks as in the case of silica polymorphs. Unlike the silica molecular sieve, silicalite, which is hydrophobic due to the absence of extra-structural cations, the aluminophosphate molecular sieves are moderately hydrophilic, apparently due to the difference in electronegativity between aluminum and phosphorus. Their intracrystalline pore volumes and pore diameters are comparable to those known for zeolites and silica molecular sieves.

In copending and commonly assigned EP-A-103 117, there is described a novel class of silicon-substituted aluminophosphates which are both microporous and crystalline. The materials have a three dimensional crystal framework of $PO_2^+$, $AlO_2^-$ and $SiO_2$ tetrahedral units and, exclusive of any alkali metal or calcium which may optionally be present, an as-synthesized empirical chemical composition on an anhydrous basis of:

mR : $(Si_xAl_yP_z)O_2$

wherein "R" represents at least one organic templating agent present in the intracrystalline pore system; "m" represents the moles of "R" present per mole of $(Si_xAl_yP_z)O_2$ and has a value of from zero to 0.3, the maximum value in each case depending upon the molecular dimensions of the templating agent and the available void volume of the pore system of the particular silicoaluminophosphate species involved; and "x", "y", and "z" represent the mole fractions of silicon, aluminum and phosphorus, respectively, present as tetrahedral oxides. The minimum value for each of "x", "y", and "z" is 0.01 and preferably 0.02. The maximum value for "x" is 0.98; for "y" is 0.60; and for "z" is 0.52. These silicoaluminophosphates exhibit several physical and chemical properties which are characteristic of aluminosilicate zeolites and aluminophosphates.

In copending and commonly assigned EP-A-121 232, there is described a novel class of titanium-containing molecular sieves whose chemical composition in the as-synthesized and anhydrous form is represented by the unit empirical formula:

mR:$(Ti_xAl_yP_z)O_2$

wherein "R" represents at least one organic templating agent present in the intracrystalline pore system;

"m" represents the moles of "R" present per mole of $(Ti_xAl_yP_z)O_2$; and has a value of between zero and about 5.0; and "x", "y" and "z" represent the mole fractions of titanium, aluminum and phosphorus, respectively, present as tetrahedral oxides.

In copending and commonly assigned EP-A-132 708, there is described a novel class of crystalline metal aluminophosphates having three-dimensional microporous framework structures of $MO_2$, $AlO_2$ and $PO_2$ tetrahedral units and having an empirical chemical composition on an anhydrous basis expressed by the formula:

$$mR:(M_xAl_yP_z)O_2$$

wherein "R" represents at least one organic templating agent present in the intracrystalline pore system; "m" represents the moles of "R" present per mole of $(M_xAl_yP_z)O_2$ and has a value of from zero to 0.3; "M" represents at least one metal of the group magnesium, manganese, zinc and cobalt; "x", "y" and "z" represent the mole fraction of the metal "M", aluminum and phosphorus, respectively, present as tetrahedral oxides.

In copending and commonly assigned EP-A-131 946, there is described a novel class of crystalline ferroaluminophosphates having a three-dimensional microporous framework structure of $FeO_2$, $AlO_2$ and $PO_2$ tetrahedral units and having an empirical chemical composition on a anhydrous basis expressed by the formula

$$mR:(Fe_xAl_yP_z)O_2$$

wherein "R" represents at least one organic templating agent present in the intracrystalline pore system; "m" represents the moles of "R" present per mole of $(Fe_xAl_yP_z)O_2$ and has a value of from zero to 0.3; "x", "y" and "z" represent the mole fraction of the iron, aluminum and phosphorus, respectively, present as tetrahedral oxides.

The instant invention relates to new molecular sieve compositions comprising at least one element, as hereinafter discussed, capable of forming a framework tetrahedral units with $AlO_2^-$ and $PO_2^+$ tetrahedral units.

## Description of the Figures

FIG. 1 is a ternary diagram wherein parameters relating to the instant compositions are set forth as mole fractions.

FIG. 2 is a ternary diagram wherein parameters relating to preferred compositions are set forth as mole fractions.

FIG. 3 is a ternary diagram wherein parameters relating to the reaction mixtures employed in the preparation of the compositions of this invention are set forth as mole fractions.

## Summary of the Invention

The instant invention relates to a new class of crystalline molecular sieves in which at least one element in addition to aluminum and phosphorus, as hereinafter defined, capable of forming a three-dimensional microporous framework of framework tetrahedral oxides to form a framework structure of $AlO_2^-$, $PO_2^+$ and $MO_2^n$ tetrahedral units wherein "M" represents at least one different element present as tetrahedral units with charge "n" where "n" may be -3, -2, -1, 0 or +1.

These new molecular sieves exhibit ion-exchange, adsorption and catalytic properties and, accordingly, find wide use as adsorbents and catalysts. The members of this novel class of compositions have crystal framework structures of $AlO_2^-$, $PO_2^+$ and tetrahedral units and have an empirical chemical composition on an anhydrous basis expressed by the formula:

$$mR : (M_xAl_yP_z)O_2$$

wherein "R" represents at least one organic templating agent present in the intracrystalline pore system; "m" represents the molar amount of "R" present per mole of $(M_xAl_yP_z)O_2$ and has a value of zero to about 0.3; "M" represents at least one element capable of forming framework tetrahedral oxides and "x", "y" and "z" represent the mole fraction of "M", aluminum and phosphorus, respectively, present as tetrahedral oxides. "M" is at least one different element such that the molecular sieves contain at least one framework tetrahedral oxide unit in addition to $AlO_2^-$ and $PO_2^+$, wherein "M" is at least one element selected from the group consisting of arsenic, beryllium, boron, chromium, gallium, germanium, lithium and vanadium.

The molecular sieves of the instant invention will be generally referred to herein by the acronym "FCAPO" to designate elements "M" in a framework of $AlO_2^-$, $PO_2^+$ and $MO_2^n$ tetrahedral oxide units. The acronym "FCAPO" denotes "Framework Constituents" of elements "M" in a framework of aluminum and phosphorus where such are all present as framework tetrahedral oxides. Actual class members will be identified by replacing the "FC" of the acronym with the element or elements present as $MO_2^n$ tetrahedral units. For example, "GeBeAPO" designates a molecular sieve comprised of $AlO_2^-$, $PO_2^+$, $GeO_2$ and $BeO_2^{-2}$ tetrahedral units.

To identify various structural species which make up each of the subgeneric classes, each species is assigned a number and is identified as "FCAPO-i" wherein "i" is an integer. The given species designation is not intended to denote a similarity in structure to any other species denominated by a similar identification system.

Detailed Description of the Invention

The instant invention relates to a new class of crystalline molecular sieves in which at least one element capable of forming framework tetrahedral oxides are provided to form a three-dimensional microporous framework structures of $AlO_2^-$, $PO_2^+$ and $MO_2^n$ tetrahedra

wherein "M" represents at least one element capable of forming tetrahedral unit, e.g., "$MO_2^n$" where "M" is an element, as discussed hereinafter, and where "n" is -3, -2, -1, 0 or +1. These new molecular sieves exhibit ion-exchange, adsorption and catalytic properties and, accordingly, find wide use as adsorbents and catalysts.

The FCAPO compositions are formed by forming a framework tetrahedral unit $MO_2^n$ in the presence of $AlO_2^-$ and $PO_2^+$ tetrahedral units where "M" is at least one element selected from the group consisting of arsenic, beryllium, boron, chromium, gallium, germanium, lithium and vanadium. In one embodiment the FCAPO compositions contain at least two of the aforementioned elements as $MO_2^n$ framework tetrahedral units.

The relative amounts of elements "M", aluminum and phosphorus are expressed by the empirical chemical formula (anhydrous):

$$mR : (M_xAl_yP_z)O_2$$

where "x", "y" and "z" represent the mole fractions of said "M", aluminum and phosphorus. The individual mole fractions of each "M" ($M_1$, $M_2$, $M_3$, etc.) may be represented by "$x_1$", "$x_2$", "$x_3$", etc. wherein "$x_1$", "$x_2$", and "$x_3$" represent the individual mole fractions of each element $M_1$, $M_2$, $M_3$, and etc. for "M" as above defined. The values of "$x_1$", "$x_2$", "$x_3$", etc. are as defined for "x" hereinbefore where "x" = $x_1$ + $x_2$ + $x_3$, etc., and where $x_1$, $x_2$, $x_3$, etc are each at least 0.01.

The molecular sieves of the instant invention comprise a framework structure of $MO_2^n$, $AlO_2^-$ and $PO_2^+$ tetrahedral units where "n" is -3, -2, -1, 0 or +1 and have an empirical chemical composition on an anhydrous basis expressed by the formula:

$$mR : (M_xAl_yP_z)O_2;$$

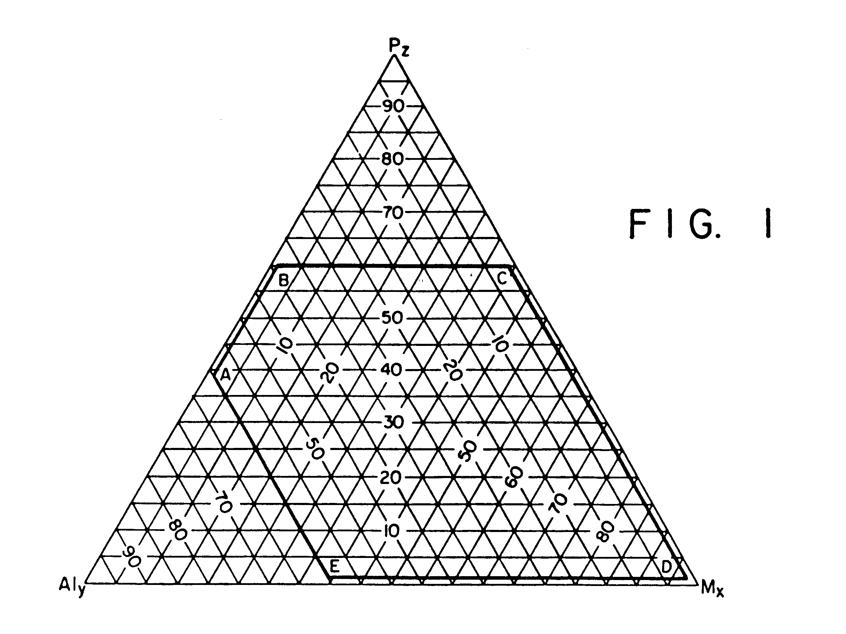

wherein "R" represents at least one organic templating agent present in the intracrystalline pore system; "m" represents the molar amount of "R" present per mole of $(M_xAl_yP_z)O_2$ and has a value of zero to about 0.3; "M" represents at least one element capable of forming framework tetrahedral oxides selected from the group consisting of arsenic, beryllium, boron, chromium, gallium, germanium, lithium and vanadium; and "x", "y" and "z" represent the mole fractions of "M", aluminum and phosphorus, respectively, present as tetrahedral oxides. The mole fractions "x", "y" and "z" are generally defined as being within the pentagonal compositional area defined by points A, B, C, D and E of the ternary diagram of FIG. 1. Points A, B, C, D and E have the following values for "x", "y" and "z":

|  | Mole Fraction | | |
|---|---|---|---|
| Point | x | y | z |
| A | 0.01 | 0.60 | 0.39 |
| B | 0.01 | 0.09 | 0.60 |
| C | 0.39 | 0.01 | 0.60 |
| D | 0.98 | 0.01 | 0.01 |
| E | 0.39 | 0.60 | 0.01 |

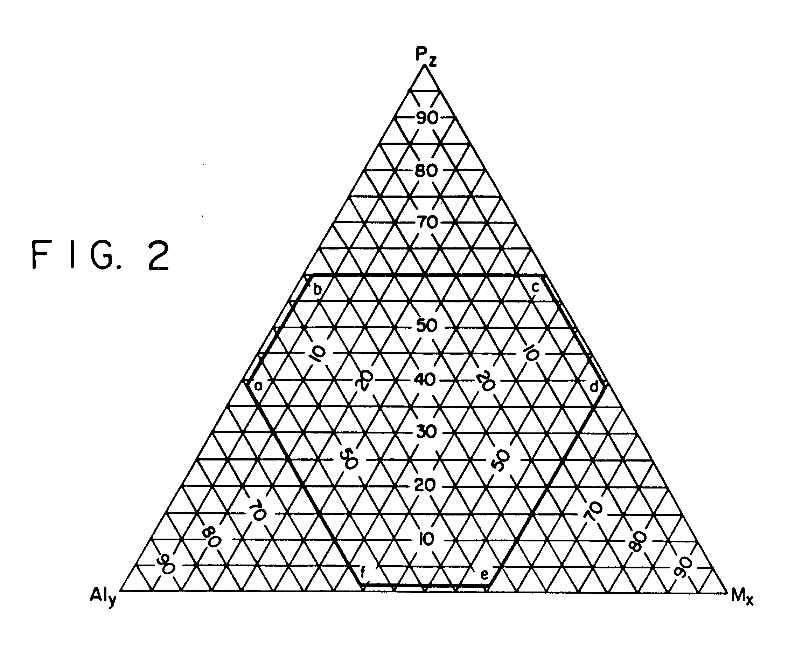

In a preferred subclass of the FCAPO molecular sieves the values of "x", "y" and "z" in the above formula are within the hexagonal compositional area defined by points a, b, c, d, e and f of the ternary diagram which is FIG. 2 of the drawings, said points a, b, c, d, e and f representing the following values for "x", "y" and "z":

|  | Mole Fraction | | |
|---|---|---|---|
| Point | x | y | z |
| a | 0.01 | 0.60 | 0.39 |
| b | 0.01 | 0.39 | 0.60 |
| c | 0.39 | 0.01 | 0.60 |
| d | 0.60 | 0.01 | 0.39 |
| e | 0.60 | 0.39 | 0.01 |
| f | 0.39 | 0.60 | 0.01 |

The FCAPOs of this invention are useful as adsorbents, catalysts, ion-exchangers, and the like in much the same fashion as aluminosilicates have been employed heretofore, although their chemical and physical properties are not necessarily similar to those observed for aluminosilicates.

FCAPO compositions are generally synthesized by hydrothermal crystallization from a reaction mixture containing active sources of elements "M", aluminum and phosphorus, preferably an organic templating, i.e., structure-directing, agent, preferably a compound of an element of Group VA of the Periodic Table, and/or optionally an alkali or other metal. The reaction mixture is generally placed in a sealed pressure vessel, preferably lined with an inert plastic material such as polytetrafluoroethylene and heated, preferably under autogenous pressure at a temperature between $50°C$ and $250°C$, and preferably between $100°C$ and $200°C$ until crystals of the FCAPO product are obtained, usually a period of from several hours to several weeks. Typical crystallization times are from about 2 hours to about 30 days with from about 4 hours to about 20 days being generally employed to obtain FCAPO products. The product is recovered by any convenient method such as centrifugation or filtration.

In synthesizing the FCAPO compositions of the instant invention, it is preferred to employ a reaction mixture composition expressed in terms of the molar ratios as follows:

$$aR : (M_xAl_yP_z)O_2 : bH_2O$$

wherein "R" is an organic templating agent; "a" is the amount of organic templating agent "R" and has a value of from zero to about 6 and is preferably an effective amount within the range of greater than zero (0) to about 6; "b" has a value of from zero (0) to about 500, preferably between about 2 and about 300; "M" represents at least one element capable of forming tetrahedral oxide framework units $MO_2^n$, with $AlO_2^-$ and $PO_2^+$ tetrahedral units where "M" is at least one element selected from the group consisting of arsenic, beryllium, boron, chromium, gallium, germanium, lithium and vanadium; "n" has a value of -3, -2, -1, 0 or +1; and "x", "y" and "z" represent the mole fractions of "M", aluminum and phosphorus, respectively, and "x", "y" and "z" have a value of at least 0.01. The value of "x" is dependent on the number of elements "M" present in the final FCAPO and is 0.01 (the minimum value of each x, e.g., $x_1$, $x_2$, $x_3$ and etc.) times the number of elements present in addition to aluminum and phosphorus and thus can range from at least 0.01 to at least 0.08 where from 1 to 8 elements may be present in addition to aluminum and phosphorus. Of course, the aforementioned amounts are minimum amounts and larger effective amounts may be employed.

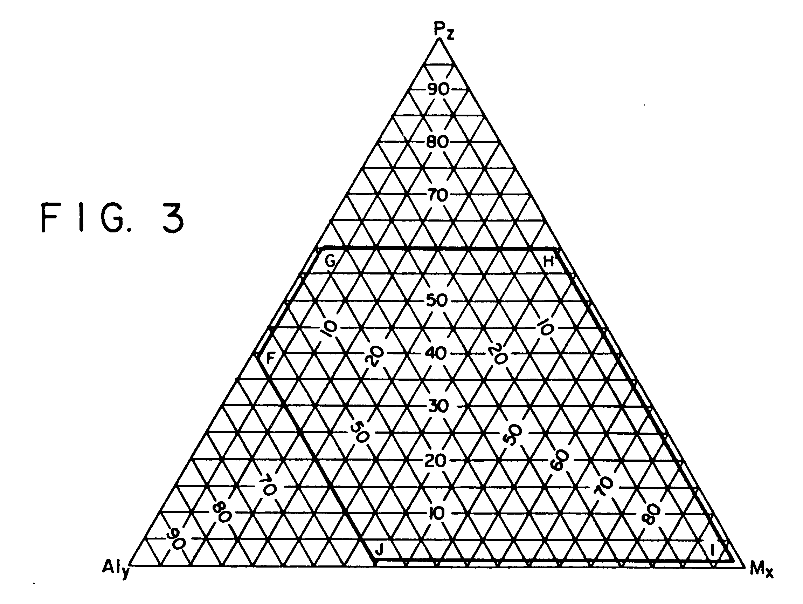

The mole fractions "x", "y" and "z" in the reaction mixture are preferably within the pentagonal compositional area defined by points F, G, H, I and J which is shown in FIG. 3 of the drawings, where points F, G, H, I and J have the following values for "x", "y" and "z":

| Point | Mole Fraction | | |
|---|---|---|---|
| | x | y | z |
| F | 0.01 | 0.60 | 0.39 |
| G | 0.01 | 0.39 | 0.60 |
| H | 0.39 | 0.01 | 0.60 |
| I | 0.98 | 0.01 | 0.01 |
| J | 0.39 | 0.60 | 0.01 |

In the foregoing expression of the reaction composition, the reactants are normalized with respect to the total of $x + y + z = 1.00$ mole, whereas in the examples the reaction mixtures are expressed in terms of the molar oxide ratios and may be normalized to 1.00 mole of $P_2O_5$. This latter form is readily converted to the former by routine calculations by dividing the total number of moles of M, aluminum and phosphorus into the moles of each of the elements "M", aluminum and phosphorus. The moles of template and water are similarly normalized by dividing by the total moles of M, aluminum and phosphorus.

In forming the reaction mixture from which the instant molecular sieves are formed the organic templating agent can be any of those heretofore proposed for use in the synthesis of conventional zeolite aluminosilicates. In general these compounds contain elements of Group VA of the Periodic Table of Elements, particularly nitrogen, phosphorus, arsenic and antimony, preferably nitrogen or phosphorus and most preferably nitrogen, which compounds also contain at least one alkyl or aryl group having from 1 to 8 carbon atoms. Particularly preferred compounds for use as templating agents are the amines, quaternary phosphonium compounds, and quaternary ammonium compounds, the latter two being represented generally by the formula $R_4X^+$, where "X" is nitrogen or phosphorus and wherein each R is an alkyl or aryl group containing from 1 to 8 carbon atoms. Polymeric quaternary ammonium salts such as $[(C_{14}H_{32}N_2)(OH)_2]_x$ wherein "x" has a value of at least 2 are also suitably employed. The mono-, di- and tri-amines are advantageously utilized, either alone or in combination with a quaternary ammonium compound or other templating compound. Mixtures of two or more templating agents can either produce mixtures of the desired FCAPOs or the more strongly directing templating species may control the course of the reaction with the other templating species serving primarily to establish the pH conditions of the reaction gel. Representative templating agents include tetramethylammonium, tetraethylammonium, tetrapropylammonium or tetrabutylammonium ions; tetrapentylammonium ions; di-n-propylamine; tripropylamine; triethylamine; triethanolamine; piperidine; cyclohexylamine; 2-methylpyridine; N,N-dimethylbenzylamine; N,N-dimethylethanolamine; choline; N,N'-dimethylpiperazine; 1,4-diaziabicyclo (2,2,2,) octane; N-methyldiethanolamine, N-methylethanolamine; N-methylpiperidine; 3-methylpiperidine; N-methylcyclohexylamine; 3-methylpyridine; 4-methylpyridine; quinuclidine; N,N'-dimethyl-1,4-diaziabicyclo (2,2,2) octane ion; di-n-butylamine, neopentylamine; di-n-pentylamine; isopropylamine; t-butylamine; ethylenediamine; pyrrolidine; and 2-imidazolidone. Not every templating agent will direct the formation of every species of FCAPO, i.e., a single templating agent can, with proper manipulation of the reaction conditions, direct the formation of several FCAPO compositions, and a given FCAPO composition can be produced using several different templating agents.

The preferred phosphorus source for the present process is phosphoric acid, but organic phosphates such as triethyl phosphate have been found satisfactory, and so also have crystalline or amorphous aluminophosphates such as the $AlPO_4$ composition of U.S.P. 4,310,440. Organo-phosphorus compounds, such as tetrabutylphosphonium bromide do not, apparently, serve as reactive sources of phosphorus, but these compounds may function as templating agents. Conventional phosphorus salts such as sodium metaphosphate, may be used, at least in part, as the phosphorus source, but are not preferred.

The preferred aluminum source is either an aluminum alkoxide, such as aluminum isoproperoxide, or pseudo-boehmite. The crystalline or amorphous aluminophosphates which are a suitable source of phosphorus are, of course, also suitable sources of aluminum. Other sources of aluminum used in zeolite synthesis, such as gibbsite, sodium aluminate and aluminum trichloride, can be employed but are not preferred.

The elements arsenic, beryllium, boron, chromium, gallium, germanium, lithium and vanadium can be introduced into the reaction system in any form which permits the formation in situ of reactive form of the element, i.e., reactive to form the framework tetrahedral unit "$MO_2^n$" of the element. Compounds which may be employed include: oxides; alkoxides; hydroxides; halides; chlorides; iodides; sulfates; bromides; organic salts such as carboxylates (e.g., acetates); nitrates; and the like.

While not essential to the synthesis of FCAPO compositions, stirring or other moderate agitation of the reaction mixture and/or seeding the reaction mixture with seed crystals of either the FCAPO species to be produced or a topologically similar aluminophosphate, aluminosilicate or molecular sieve composition, facilitates the crystallization procedure.

After crystallization the FCAPO product may be isolated and advantageously washed with water and dried in air. The as-synthesized FCAPO generally contains within its internal pore system at least one form of the templating agent employed in its formation. Most commonly the organic moiety is present, at least in part, as a charge-balancing cation as is generally the case with as-synthesized aluminosilicate zeolites prepared from organic-containing reaction systems. It is possible, however, that some or all of the organic moiety is an occluded molecular species in a particular FCAPO species. As a general rule the templating agent, and hence the occluded organic species, is too large to move freely through the pore system of the FCAPO product and must be removed by calcining the FCAPO at temperatures of $200°C$ to $700°C$ to thermally degrade the organic species. In a few instances the pores of the FCAPO product are sufficiently large to permit transport of the templating agent, particularly if the latter is a small molecule, and accordingly complete or partial removal thereof can be accomplished by conventional desorption procedures such as carried out in the case of zeolites. It will be understood that the term "as-synthesized" as used herein does not include the condition of the FCAPO phase wherein the organic moiety occupying the intracrystalline pore system as a result of the hydrothermal crystallization process has been reduced by post-synthesis treatment such that the value of "m" in the composition formula

$$mR : (M_xAl_yP_z)O_2$$

has a value of less than 0.02. The other symbols of the formula are as defined hereinabove. In those preparations in which an aluminum alkoxide is employed as the source of "M", aluminum or phosphorus, the corresponding alcohol is necessarily present in the reaction mixture since it is a hydrolysis product of the alkoxide. It has not been determined whether this alcohol participates in the synthesis process as a templating agent. For the purposes of this application, however, this alcohol is arbitrarily omitted from the class of templating agents, even if it is present in the as-synthesized FCAPO material.

Since the present FCAPO compositions are formed from $MO_2^n$, $AlO_2$, and $PO_2$ tetrahedral units which, respectively, have a net charge of "n", -1 and +1, where "n" is -3, -2, -1, 0 or +1, the matter of cation exchangeability is considerably more complicated than in the case of zeolitic molecular sieves in which, ideally, there is a stoichiometric relationship between $AlO_2^-$ tetrahedra and charge-balancing cations. In the instant compositions, an $AlO_2^-$ tetrahedron can be balanced electrically either by association with a $PO_2^+$ tetrahedron or a simple cation such as an alkali metal cation, a proton ($H^+$), a cation of "M" present in the reaction mixture, or an organic cation derived from the templating agent. Similarly a $MO_2^n$ tetrahedron can be balanced electrically by association with $PO_2^+$ or $AlO_2^-$ tetrahedra, a cation of "M" present in the reaction mixture, a simple cation unless such as an alkali metal cation, a proton ($H^+$), organic cations derived from the templating agent, or other divalent or polyvalent metal anions or cations introduced from an extraneous source. It has also been postulated that non-adjacent $AlO_2^-$ and $PO_2^+$ tetrahedral pairs can be balanced by $Na^+$ and $OH^-$ respectively [Flanigen and Grose, Molecular Sieve Zeolites-I, ACS, Washington, DC (1971)].

The FCAPO compositions of the present invention may exhibit cation-exchange capacity when analyzed using ion-exchange techniques heretofore employed with zeolitic aluminosilicates and have pore diameters which are inherent in the lattice structure of each species and which are at least about 3Å in diameter. Ion exchange of FCAPO compositions is ordinarily possible only after the organic moiety derived from the template, present as a result of synthesis, has been removed from the pore system. Dehydration to remove water present in the as-synthesized FCAPO compositions can usually be accomplished, to some degree at least, in the usual manner without removal of the organic moiety, but the absence of the organic species greatly facilitates adsorption and desorption procedures. As illustrated hereinafter, the FCAPO materials have various degrees of hydrothermal and thermal stability, some being quite remarkable in this regard, and function well as molecular sieve adsorbents and hydrocarbon conversion catalysts or catalyst bases.

In preparing the FCAPO compositions it is preferred to use a stainless steel reaction vessel utilized lined with an inert plastic material, polytetrafluoroethylene, to avoid contamination of the reaction mixture. In general, the final reaction mixture from which each FCAPO composition is crystallized is prepared by forming mixtures of less than all of the reagents and thereafter incorporating into these mixtures additional reagents either singly or in the form of other intermediate mixtures of two or more reagents. In some instances the reagents admixed retain their identity in the intermediate mixture and in other cases some or all of the reagents are involved in chemical reactions to produce new reagents. The term "mixture" is applied in both cases. Further, unless otherwise specified, each intermediate mixture as well as the final reaction mixture was stirred until substantially homogeneous.

X-ray patterns of reaction products are obtained by X-ray analysis using standard X-ray powder diffraction techniques. The radiation source is a high-intensity, copper target, X-ray tube operated at 50 kV and 40 mA. The diffraction pattern from the copper K-alpha radiation and graphite monochromator is suitably recorded by an X-ray spectrometer scintillation counter, pulse height analyzer and strip chart recorder. Flat compressed powder samples are scanned at 2° (2 theta) per minute, using a two second time constant. Interplanar spacings (d) in Angstrom units are obtained from the position of the diffraction peaks expressed as $2\theta$ where $\theta$ is the Bragg angle as observed on the strip chart. Intensities are determined from the heights of diffraction peaks after subtracting background, "$I_o$" being the intensity of the strongest line or peak, and "I" being the intensity of each of the other peaks. Alternatively the X-ray analysis may be carried out using copper K-alpha radiation with Siemens Type K-805 X-ray sources and Siemens D-500 X-ray powder diffractometers, available from Siemens Corporation, Cherry Hill, NJ.

As will be understood by those skilled in the art the determination of the parameter 2 theta is subject to both human and mechanical error, which in combination, can impose an uncertainty of about ±0.4° on each reported value of 2 theta. This uncertainty is, of course, also manifested in the reported values of the d-spacings, which are calculated from the 2 theta values. This imprecision is general throughout the art and is not sufficient to preclude the differentiation of the present crystalline materials from each other and from the compositions of the prior art. In some of the X-ray patterns reported, the relative intensities of the d-spacings are indicated by the notations vs, s, m, w and vw which represent very strong, strong, medium, weak and very weak respectively.

In certain instances, the purity of a synthesized product may be assessed with reference to its X-ray powder diffraction pattern. Thus, for example, if a sample is stated to be pure, it is intended only that the X-ray pattern of the sample is free of lines attributable to crystalline impurities, not that there are no amorphous materials present.

The molecular sieves of the instant invention may be characterized by their x-ray powder diffraction patterns and such may have one of the x-ray patterns set forth in the following Tables A through V, wherein said x-ray patterns are for both the as-synthesized and calcined forms unless otherwise noted:

TABLE A (FCAPO-5)

| $2\theta$ | $d(nm) \cdot 10^{-1}$ | Relative Intensity |
|---|---|---|
| 7.3 - 7.65 | 12.1 - 11.56 | m - vs |
| 19.5 - 19.95 | 4.55 - 4.46 | m - s |
| 20.9 - 21.3 | 4.25 - 4.17 | m - vs |
| 22.2 - 22.6 | 4.00 - 3.93 | w - vs |
| 25.7 - 26.15 | 3.47 - 3.40 | w - m |

TABLE B (FCAPO-11)

| $2\theta$ | $d(nm) \cdot 10^{-1}$ | Relative Intensity |
|---|---|---|
| 9.3 - 9.65 | 9.51 - 9.17 | m - s |
| 20.2 - 20.6 | 4.40 - 4.31 | m - s |
| 20.9 - 21.3 | 4.25 - 4.17 | s - vs |
| 22.0 - 22.5 | 4.04 - 3.95 | m - s |
| 22.5 - 22.9 | 3.95 - 3.92 | m - s |
| 23.0 - 23.4 | 3.87 - 3.80 | m - vs |

## TABLE C (FCAPO-14)

| $2\theta$ | $d(nm) \cdot 10^{-1}$ | Relative Intensity |
|---|---|---|
| 8.6 - 8.9 | 10.3 - 9.93 | vs |
| 13.0 | 6.81 | w |
| 21.9 - 22.2 | 4.06 - 4.00 | w |
| 25.4 | 3.51 | w |
| 27.5 | 3.24 | w |
| 29.7 | 3.01 | w |

## TABLE D (FCAPO-16)

| $2\theta$ | $d(nm) \cdot 10^{-1}$ | Relative Intensity |
|---|---|---|
| 11.3 - 11.6 | 7.83 - 7.63 | m - vs |
| 18.7 - 18.9 | 4.75 - 4.70 | w - s |
| 21.9 - 22.3 | 4.06 - 3.99 | m - vs |
| 26.5 - 27.0 | 3.363 - 3.302 | w - m |
| 29.7 - 30.05 | 3.008 - 2.974 | w - m |

## TABLE E (FCAPO-17)

| $2\theta$ | $d(nm) \cdot 10^{-1}$ | Relative Intensity |
|---|---|---|
| 7.7 - 7.75 | 11.5 - 11.4 | vs |
| 13.4 | 6.61 | s - vs |
| 15.5 - 15.55 | 5.72 - 5.70 | s |
| 19.65 - 19.7 | 4.52 - 4.51 | w - s |
| 20.5 - 20.6 | 4.33 - 4.31 | vs |
| 31.8 - 32.00 | 2.812 - 2.797 | w - s |

## TABLE F (FCAPO-18)

| $2\theta$ | $d(nm) \cdot 10^{-1}$ | Relative Intensity |
|---|---|---|
| 9.6 - 9.65 | 9.21 - 9.16 | vs |
| 15.5 - 15.55 | 5.72 - 5.70 | m |
| 16.9 - 17.1 | 5.25 - 5.19 | m |
| 20.15 - 20.25 | 4.41 - 4.39 | m |
| 20.95 - 21.05 | 4.24 - 4.22 | m |
| 31.8 - 32.5 | 2.814 - 2.755 | m |

**EP 0 158 976 B1**

### TABLE G (FCAPO-20)

| $2\theta$ | $d(nm) \cdot 10^{-1}$ | Relative Intensity |
|---|---|---|
| 13.7 - 14.25 | 6.46 - 6.22 | m - vs |
| 19.55 - 20.0 | 4.54 - 4.44 | w - s |
| 24.05 - 24.5 | 3.70 - 3.63 | m - vs |
| 34.3 - 35.0 | 2.614 - 2.564 | vw - w |
| 42.5 - 43.0 | 2.127 - 2.103 | vw - w |

### TABLE H (FCAPO-31)

| $2\theta$ | $d(nm) \cdot 10^{-1}$ | Relative Intensity |
|---|---|---|
| 8.5 - 8.6 | 10.40 - 10.28 | m - s |
| 20.2 - 20.3 | 4.40 - 4.37 | m |
| 21.9 - 22.1 | 4.06 - 4.02 | w - m |
| 22.6 - 22.7 | 3.93 - 3.92 | vs |
| 31.7 - 31.8 | 2.823 - 2.814 | w - m |

### TABLE J* (FCAPO-33)

| $2\theta$ | $d(nm) \cdot 10^{-1}$ | Relative Intensity |
|---|---|---|
| 9.25 - 9.55 | 9.56 - 9.26 | w - m |
| 12.5 - 12.9 | 7.08 - 6.86 | vs |
| 16.9 - 17.3 | 5.25 - 5.13 | w - m |
| 20.45 - 20.9 | 4.34 - 4.25 | w - m |
| 23.85 - 24.25 | 3.73 - 3.67 | w - m |
| 26.05 - 26.35 | 3.42 - 3.38 | w - m |
| 27.3 - 27.6 | 3.27 - 3.23 | vs |

* as-synthesized form

### TABLE K* (FCAPO-33)

| $2\theta$ | $d(nm) \cdot 10^{-1}$ | Relative Intensity |
|---|---|---|
| 13.15 - 13.4 | 6.73 - 6.61 | vs |
| 18.05 - 18.35 | 4.91 - 4.83 | m |
| 18.4 - 18.6 | 4.82 - 4.77 | m |
| 26.55 - 26.7 | 3.36 - 3.34 | m |
| 32.0 - 32.1 | 2.80 - 2.79 | m |

* calcined form

### TABLE L (FCAPO-34)

| $2\theta$ | $d(nm) \cdot 10^{-1}$ | Relative Intensity |
|---|---|---|
| 9.4 - 9.65 | 9.41 - 9.17 | s - vs |
| 15.9 - 16.2 | 5.57 - 5.47 | vw - m |
| 17.85 - 18.4 | 4.97 - 4.82 | w - s |
| 20.3 - 20.9 | 4.37 - 4.25 | m - vs |
| 24.95 - 25.4 | 3.57 - 3.51 | vw - s |
| 30.3 - 30.8 | 2.95 - 2.90 | w - s |

### TABLE M (FCAPO-35)

| $2\theta$ | $d(nm) \cdot 10^{-1}$ | Relative Intensity |
|---|---|---|
| 10.8 - 11.1 | 8.19 - 7.97 | m |
| 17.2 - 17.4 | 5.16 - 5.10 | s - vs |
| 21.0 - 21.25 | 4.23 - 4.18 | m - s |
| 21.8 - 22.0 | 4.08 - 4.04 | vs |
| 31.8 - 32.2 | 2.814 - 2.788 | m |

### TABLE N (FCAPO-36)

| $2\theta$ | $d(nm) \cdot 10^{-1}$ | Relative Intensity |
|---|---|---|
| 7.7 - 7.9 | 11.5 - 11.2 | vs |
| 16.2 - 16.6 | 5.47 - 5.34 | w - m |
| 18.9 - 19. 3 | 4.70 - 4.60 | m - s |
| 20.6 - 20.8 | 4.31 - 4.27 | w - s |
| 21.8 - 22.0 | 4.08 - 4.04 | m |
| 22.2 - 22.5 | 4.00 - 3.95 | w - m |

### TABLE O (FCAPO-37)

| $2\theta$ | $d(nm) \cdot 10^{-1}$ | Relative Intensity |
|---|---|---|
| 6.1 - 6.3 | 14.49 - 14.03 | vs |
| 15.5 - 15.7 | 5.72 - 5.64 | w - m |
| 18.5 - 18.8 | 4.80 - 4.72 | w - m |
| 23.5 - 23.7 | 3.79 - 3.75 | w - m |
| 26.9 - 27.1 | 3.31 - 3.29 | w - m |

TABLE P (FCAPO-39)

| $2\theta$ | $d(nm) \cdot 10^{-1}$ | Relative Intensity |
|---|---|---|
| 9.4 - 9.6 | 9.41 - 9.21 | w - m |
| 13.3 - 13.6 | 6.66 - 6.51 | m - vs |
| 18.0 - 18.4 | 4.93 - 4.82 | m |
| 21.2 - 21.5 | 4.19 - 4.13 | m - s |
| 22.5 - 23.0 | 3.95 - 3.87 | s - vs |
| 30.2 - 30.5 | 2.96 - 2.93 | w - m |

TABLE Q (FCAPO-40)

| $2\theta$ | $d(nm) \cdot 10^{-1}$ | Relative Intensity |
|---|---|---|
| 7.5 - 7.7 | 11.79 - 11.48 | vw - m |
| 8.0 - 8.1 | 11.05 - 10.94 | s - vs |
| 12.4 - 12.5 | 7.14 - 7.08 | w - vs |
| 13.6 - 13.8 | 6.51 - 6.42 | m - s |
| 14.0 - 14.1 | 6.33 - 6.28 | w - m |
| 27.8 - 28.0 | 3.209 - 3.187 | w - m |

TABLE R (FCAPO-41)

| $2\theta$ | $d(nm) \cdot 10^{-1}$ | Relative Intensity |
|---|---|---|
| 13.6 - 13.8 | 6.51 - 6.42 | w - m |
| 20.5 - 20.6 | 4.33 - 4.31 | w - m |
| 21.1 - 21.3 | 4.21 - 4.17 | vs |
| 22.1 - 22.3 | 4.02 - 3.99 | m - s |
| 22.8 - 23.0 | 3.90 - 3.86 | m |
| 23.1 - 23.4 | 3.82 - 3.80 | w - m |
| 25.5 - 25.9 | 3.493 - 3.440 | w - m |

TABLE S (FCAPO-42)

| $2\theta$ | $d(nm) \cdot 10^{-1}$ | Relative Intensity |
|---|---|---|
| 7.15 - 7.4 | 12.36 - 11.95 | m - vs |
| 12.5 - 12.7 | 7.08 - 6.97 | m - s |
| 21.75 - 21.9 | 4.09 - 4.06 | m - s |
| 24.1 - 24.25 | 3.69 - 3.67 | vs |
| 27.25 - 27.4 | 3.273 - 3.255 | s |
| 30.05 - 30.25 | 2.974 - 2.955 | m - s |

TABLE T (FCAPO-44)

| $2\theta$ | $d(nm) \cdot 10^{-1}$ | Relative Intensity |
|---|---|---|
| 9.4 - 9.55 | 9.41 - 9.26 | vs |
| 13.0 - 13.1 | 6.81 - 6.76 | w - m |
| 16.0 - 16.2 | 5.54 - 5.47 | w - m |
| 20.6 - 20.85 | 4.31 - 4.26 | s - vs |
| 24.3 - 24.4 | 3.66 - 3.65 | w - vs |
| 30.7 - 30.95 | 2.912 - 2.889 | w - s |

TABLE U (FCAPO-46)

| $2\theta$ | $d(nm) \cdot 10^{-1}$ | Relative Intensity |
|---|---|---|
| 7.2 - 8.1 | 12.3 - 10.9 | vs |
| 21.2 - 21.8 | 4.19 - 4.08 | w - m |
| 22.5 - 23.0 | 3.95 - 3.87 | vw - m |
| 26.6 - 27.2 | 3.351 - 3.278 | vw - w |
| 28.5 - 29.0 | 3.132 - 3.079 | vw - w |

TABLE V (FCAPO-47)

| $2\theta$ | $d(nm) \cdot 10^{-1}$ | Relative Intensity |
|---|---|---|
| 9.4 | 9.41 | vs |
| 15.9 - 16.0 | 5.57 - 5.54 | w - m |
| 20.5 - 20.6 | 4.33 - 4.31 | s |
| 24.5 - 24.7 | 3.63 - 3.60 | w |
| 25.8 - 25.9 | 3.45 - 3.44 | w |
| 30.4 - 30.5 | 2.940 - 2.931 | w |

The following examples are provided to further illustrate the invention and are not intended to be limiting thereof:

Example 1 (Preparation of AsAPO-5)

a) AsAPO-5 is prepared from a reaction mixture having a composition, expressed in terms of the molar oxide ratios of the components of the reaction mixture, of:

1.0-2.0 TPA : 0.05-0.2 $As_2O_q$ : 0.5-1.0 $Al_2O_3$ : 0.5-1.0 $P_2O_5$ : 40-100 $H_2O$

where "TPA" denotes tripropylamine and "q" denotes the oxidation state of arsenic.

The reaction mixture is digested by placing the reaction mixture in a sealed stainless steel pressure vessel and heating it at an effective temperature and for an effective time until crystals of the AsAPO-5 product are obtained. Solids are recovered by filtration, washed with water and dried in air at room temperature.

The AsAPO-5 product's chemical analysis shows the AsAPO-5 product contains arsenic, aluminum and phosphorus in amounts within the hexagonal compositional area defined by points a, b, c, d, e and f of FIG.2.

The x-ray powder diffraction pattern of an AsAPO-5 product is characterized by the following data:

| $2\theta$ | d(nm)$\cdot 10^{-1}$ | Relative Intensity |
|---|---|---|
| 7.3 - 7.65 | 12.1 - 11.56 | m - vs |
| 19.5 - 19.95 | 4.55 - 4.46 | m - s |
| 20.9 - 21.3 | 4.25 - 4.17 | m - vs |
| 22.2 - 22.6 | 4.00 - 3.93 | w - vs |
| 25.7 - 26.15 | 3.47 - 3.40 | w - m |

b) The x-ray powder diffraction pattern for a calcined AsAPO-5 is also characterized by the X-ray pattern of part a).

c) When the calcined AsAPO-5 of part (b) is utilized in adsorption capacity studies using a standard McBain-Bakr gravimetric adsorption apparatus the measurements are made on a sample after activation at 350$^{\circ}$C in a vacuum. The following data are used in the adsorption studies:

| Adsorbate | Kinetic Diameter (nm)$\cdot 10^{-1}$ | Pressure (mbar) (Torr) | Temp, $^{\circ}$C | Wt. % Adsorbed* |
|---|---|---|---|---|
| $O_2$ | 3.46 | 133 (100) | -183 | 7 |
| $O_2$ | 3.46 | 1000 (750) | -183 | 10 |
| Neopentane | 6.2 | 933 (700) | 24 | 4 |
| $H_2O$ | 2.65 | 5.7 (4.3) | 24 | 4 |
| $H_2O$ | 2.65 | 27 (20.0) | 24 | 12 |

*typical amount adsorbed

The pore diameter of AsAPO-5 is greater than 0,62 nm (6.2 Å).

Example 2 (Preparation of AsAPO-11)

a) AsAPO-11 is prepared from a reaction mixture having a composition, expressed in terms of the molar oxide ratios of the components of the reaction mixture, of:

1.0-2.0 DPA : 0.05-0.2 $As_2O_q$ : 0.5-1.0 $Al_2O_3$ : 0.5-1.0 $P_2O_5$ : 40-100 $H_2O$

where "DPA" denotes di-n-propylamine and "q" denotes the oxidation state of arsenic.

The reaction mixture is digested by placing the reaction mixture in a sealed stainless steel pressure vessel and heating it at an effective temperature and for an effective time until crystals of the AsAPO-11 product are obtained. Solids are then recovered by filtration, washed with water and dried in air at room temperature.

The AsAPO-11 product's chemical analysis shows the AsAPO-11 product contains arsenic, aluminum and phosphorus in amounts within the hexagonal compositional area defined by points a, b, c, d, e and f of FIG.2.

The x-ray powder diffraction pattern of an AsAPO-11 product is characterized by the following data:

| $2\theta$ | d(nm)$\cdot 10^{-1}$ | Relative Intensity |
|---|---|---|
| 9.3 - 9.65 | 9.51 - 9.17 | m - s |
| 20.2 - 20.6 | 4.40 - 4.31 | m - s |
| 20.9 - 21.3 | 4.25 - 4.17 | s - vs |
| 22.0 - 22.5 | 4.04 - 3.95 | m - s |
| 22.5 - 22.9 | 3.95 - 3.92 | m - s |
| 23.0 - 23.4 | 3.87 - 3.80 | m - vs |

b) The x-ray powder diffraction pattern for a calcined AsAPO-11 is also characterized by the X-ray pattern of part a).

c) When the calcined AsAPO-11 of part (b) is utilized in adsorption capacity studies using a standard McBain-Bakr gravimetric adsorption apparatus the measurements are made on a sample after activation at 350$^{\circ}$C in a vacuum. The following data are used in the adsorption studies:

| Adsorbate | Kinetic Diameter $(nm) \cdot 10^{-1}$ | Pressure (mbar)(Torr) | Temp, $^\circ$C | Wt. % Adsorbed* |
|---|---|---|---|---|
| $O_2$ | 3.46 | 133(100) | -183 | 5 |
| $O_2$ | 3.46 | 1000(750) | -183 | 6 |
| Cyclohexane | 6.0 | 120(90) | 24 | 4 |
| $H_2O$ | 2.65 | 5.7(4.3) | 24 | 6 |
| $H_2O$ | 2.65 | 27(20) | 24 | 8 |

*typical amount adsorbed

The pore diameter of AsAPO-11 is about 0,6 nm (6Å).

Example 3 (Preparation of BAPO-5)

a) BAPO-5 is prepared from a reaction mixture having a composition, expressed in terms of the molar oxide ratios of the components of the reaction mixture, of:

1.0-2.0 TPA : 0.05-0.2 $B_2O_3$ : 0.5-1.0 $Al_2O_3$ : 0.5-1.0 $P_2O_5$ : 40-100 $H_2O$

where "TPA" denotes tripropylamine.

The reaction mixture is digested by placing the reaction mixture in a sealed stainless steel pressure vessel and heating it at an effective temperature and for an effective time hours to produce the BAPO-5 product. Solids are recovered by filtration, washed with water and dried in air at room temperature.

The BAPO-5 product's chemical analysis shows the BAPO-5 product contains boron, aluminum and phosphorus in amounts within the hexagonal compositional area defined by points a, b, c, d, e and f of FIG.2.

The x-ray powder diffraction pattern of a BAPO-5 product is characterized by the following data:

| $2\theta$ | $d(nm) \cdot 10^{-1}$ | Relative Intensity |
|---|---|---|
| 7.3 - 7.65 | 12.1 - 11.56 | m - vs |
| 19.5 - 19.95 | 4.55 - 4.46 | m - s |
| 20.9 - 21.3 | 4.25 - 4.17 | m - vs |
| 22.2 - 22.6 | 4.00 - 3.93 | w - vs |
| 25.7 - 26.15 | 3.47 - 3.40 | w - m |

b) The x-ray powder diffraction pattern for a calcined BAPO-5 is also characterized by the X-ray pattern of part a).

c) When the calcined BAPO-5 of part (b) is utilized in adsorption capacity studies using a standard McBain-Bakr gravimetric adsorption apparatus the measurements are made on a sample after activation at 350$^\circ$C in vacuum. The following data are used in the adsorption studies:

| Adsorbate | Kinetic Diameter$(nm) \cdot 10^{-1}$ | Pressure (mbar)(Torr) | Temp, $^\circ$C | Wt.% Adsorbed* |
|---|---|---|---|---|
| $O_2$ | 3.46 | 133 (100) | -183 | 7 |
| $O_2$ | 3.46 | 1000 (750) | -183 | 10 |
| Neopentane | 6.2 | 933 (700) | 24 | 4 |
| $H_2O$ | 2.65 | 5.7 (4.3) | 24 | 4 |
| $H_2O$ | 2.65 | 27 (20.0) | 24 | 12 |

*typical amount adsorbed

The pore diameter of BAPO-5 is greater than 0,62 nm (6.2 Å).

Example 4 (Preparation of BAPO-11)

a) BAPO-11 is prepared from a reaction mixture having a composition, expressed in terms of the molar oxide ratios of the components of the reaction mixture, of:

1.0-2.0 DPA : 0.05-0.2 $B_2O_3$ : 0.5-1.0 $Al_2O_3$ : 0.5-1.0 $P_2O_5$ : 40-100 $H_2O$

where "DPA" denotes di-n-propylamine.

The reaction mixture is digested by placing the reaction mixture in a sealed stainless steel pressure vessel and heating it at an effective temperature and for an effective time to produce the BAPO-11 product. Solids are then recovered by filtration, washed with water and dried in air at room temperature.

The BAPO-11 product's chemical analysis shows the BAPO-11 product contains boron, aluminum and phosphorus in amounts within the hexagonal compositional area defined by points a, b, c, d, e and f of FIG.2.

The x-ray powder diffraction pattern of a BAPO-11 product is characterized by the following data:

| $2\theta$ | d(nm)•$10^{-1}$ | Relative Intensity |
|---|---|---|
| 9.3 - 9.65 | 9.51 - 9.17 | m - s |
| 20.2 - 20.6 | 4.40 - 4.31 | m - s |
| 20.9 - 21.3 | 4.25 - 4.17 | s - vs |
| 22.0 - 22.5 | 4.04 - 3.95 | m - s |
| 22.5 - 22.9 | 3.95 - 3.92 | m - s |
| 23.0 - 23.4 | 3.87 - 3.80 | m - vs |

b) The x-ray powder diffraction pattern for a calcined BAPO-11 is also characterized by the pattern of part a).

c) When the calcined BAPO-11 of part (b) is utilized in adsorption capacity studies using a standard McBain-Bakr gravimetric adsorption apparatus the measurements are made on a sample after activation at 350˚ C in vacuum. The following data are used in the adsorption studies:

| Adsorbate | Kinetic Diameter (nm)•$10^{-1}$ | Pressure (mbar) (Torr) | Temp, ˚ C | Wt. % Adsorbed* |
|---|---|---|---|---|
| $O_2$ | 3.46 | 133 (100) | -183 | 5 |
| $O_2$ | 3.46 | 1000 (750) | -183 | 6 |
| Cyclohexane | 6.0 | 120 (90) | 24 | 4 |
| $H_2O$ | 2.65 | 5.7 (4.3) | 24 | 6 |
| $H_2O$ | 2.65 | 27 (20) | 24 | 8 |

*typical amount adsorbed

The pore diameter of BAPO-11 is about 0,6 nm (6Å).

Example 5 (Preparation of BeAPO-5)

a) BeAPO-5 is prepared from a reaction mixture having a composition, expressed in terms of the molar oxide ratios of the components of the reaction mixture, of:

1.0-2.0 TPA : 0.1-0.4 BeO : 0.5-1.0 $Al_2O_3$ : 0.5-1.0 $P_2O_5$ : 40-100 $H_2O$

where "TPA" denotes tripropylamine.

The reaction mixture is digested by placing the reaction mixture in a sealed stainless steel pressure vessel and heating it at an effective temperature and for an effective time until crystals of the BeAPO-5 product are obtained. Solids are recovered by filtration, washed with water and dried in air at room temperature.

The BeAPO-5 product's chemical analysis shows the BeAPO-5 product contains beryllium, aluminum and phosphorus in amounts within the hexagonal compositional area defined by points a, b, c, d, e and f of FIG.2.

The x-ray powder diffraction pattern of a BeAPO-5 product is characterized by the following data:

| $2\theta$ | $d(nm) \cdot 10^{-1}$ | Relative Intensity |
|---|---|---|
| 7.3 - 7.65 | 12.1 - 11.56 | m - vs |
| 19.5 - 19.95 | 4.55 - 4.46 | m - s |
| 20.9 - 21.3 | 4.25 - 4.17 | m - vs |
| 22.2 - 22.6 | 4.00 - 3.93 | w - vs |
| 25.7 - 26.15 | 3.47 - 3.40 | w - m |

b) The x-ray powder diffraction pattern for a calcined BeAPO-5 is also characterized by the x-ray pattern of part a).

c) When the calcined BeAPO-5 of part (b) is utilized in adsorption capacity studies using a standard McBain-Bakr gravimetric adsorption apparatus the measurements are made on a sample after activation at 350° C in a vacuum. The following data are used in the adsorption studies:

| Adsorbate | Kinetic Diameter $(nm) \cdot 10^{-1}$ | Pressure (mbar) (Torr) | Temp, °C | Wt. % Adsorbed* |
|---|---|---|---|---|
| $O_2$ | 3.46 | 133 (100) | -183 | 7 |
| $O_2$ | 3.46 | 1000 (750) | -183 | 10 |
| Neopentane | 6.2 | 933 (700) | 24 | 4 |
| $H_2O$ | 2.65 | 5.7 (4.3) | 24 | 4 |
| $H_2O$ | 2.65 | 27 (20.0) | 24 | 12 |

*typical amount adsorbed

The pore diameter of BeAPO-5 is greater than 0,62 nm (6.2 Å).

Example 6 (Preparation of BeAPO-17)

a) BeAPO-17 is prepared from a reaction mixture having a composition, expressed in terms of the molar oxide ratios of the components of the reaction mixture, of:

1.0-2.0 QN : 0.1-0.4 BeO : 0.5-1.0 $Al_2O_3$ : 0.5-1.0 $P_2O_5$ : 40-100 $H_2O$

where "QN" denotes quinuclidine.

The reaction mixture is digested by placing the reaction mixture in a sealed stainless steel pressure vessel and heating it at an effective temperature and for an effective time until crystals of the BeAPO-17 product are obtained. Solids are then recovered by filtration, washed with water and dried in air at room temperature.

The BeAPO-17 product's chemical analysis shows the BeAPO-17 product contains beryllium, aluminum and phosphorus in amounts within the hexagonal compositional area defined by points a, b, c, d, e and f of FIG.2.

The x-ray powder diffraction pattern of a BeAPO-17 product is characterized by the following data:

| $2\theta$ | $d(nm) \cdot 10^{-1}$ | Relative Intensity |
|---|---|---|
| 7.7 - 7.75 | 11.5 - 11.4 | vs |
| 13.4 | 6.61 | s - vs |
| 15.5 - 15.55 | 5.72 - 5.70 | s |
| 19.65 - 19.7 | 4.52 - 4.51 | w - s |
| 20.5 - 20.6 | 4.33 - 4.31 | vs |
| 31.8 - 32.00 | 2.812 - 2.797 | w - s |

b) The x-ray powder diffraction pattern for a calcined BeAPO-17 is also characterized by the x-ray pattern of part a).

c) When the calcined BeAPO-17 of part (b) is utilized in adsorption capacity studies using a standard McBain-Bakr gravimetric adsorption apparatus the measurements are made on a sample after activation at 350° C in a vacuum. The following data are used in the adsorption studies:

| Adsorbate | Kinetic Diameter (nm)$\cdot 10^{-1}$ | Pressure (mbar) (Torr) | Temp $^\circ$C | Wt. % Adsorbed* |
|---|---|---|---|---|
| $O_2$ | 3.46 | 133 (100) | -183 | 10 |
| $O_2$ | 3.46 | 1000 (750) | -183 | 12 |
| n-butane | 4.3 | 133 (100) | 24 | 4 |
| $H_2O$ | 2.65 | 5.7 (4.3) | 24 | 13 |
| $H_2O$ | 2.65 | 27 (20) | 24 | 14 |

*typical amount adsorbed

The pore diameter of BeAPO-17 is about 0,43 nm (4.3 Å).

Example 7 (Preparation of CAPO-5)

a) CAPO-5 is prepared from a reaction mixture having a composition, expressed in terms of the molar oxide ratios of the components of the reaction mixture, of:

1.0-2.0 TPA : 0.05-0.2 $Cr_2O_q$ : 0.5-1.0 $Al_2O_3$ : 0.5-1.0 $P_2O_5$ : 40-100 $H_2O$

where "TPA" denotes tripropylamine, and "q" denotes the oxidation state of chromium.

The reaction mixture is digested by placing the reaction mixture in a sealed stainless steel pressure vessel and heating it at an effective temperature and for an effective time until crystals of the CAPO-5 product are obtained. Solids are recovered by filtration, washed with water and dried in air at room temperature.

The CAPO-5 product's chemical analysis shows the CAPO-5 product contains chromium, aluminum and phosphorus in amounts within the hexagonal compositional area defined by points a, b, c, d, e and f of FIG.2.

The x-ray powder diffraction pattern of a CAPO-5 product is characterized by the following data:

| $2\theta$ | d(nm)$\cdot 10^{-1}$ | Relative Intensity |
|---|---|---|
| 7.3 - 7.65 | 12.1 - 11.56 | m - vs |
| 19.5 - 19.95 | 4.55 - 4.46 | m - s |
| 20.9 - 21.3 | 4.25 - 4.17 | m - vs |
| 22.2 - 22.6 | 4.00 - 3.93 | w - vs |
| 25.7 - 26.15 | 3.47 - 3.40 | w - m |

b) The x-ray powder diffraction pattern for a calcined CAPO-5 is also characterized by the X-ray pattern of part a).

c) When the calcined CAPO-5 of part (b) is utilized in adsorption capacity studies using a standard McBain-Bakr gravimetric adsorption apparatus the measurements are made on a sample after activation at 350 $^\circ$C in a vacuum. The following data are used in the adsorption studies:

| Adsorbate | Kinetic Diameter (nm)$\cdot 10^{-1}$ | Pressure (mbar) (Torr) | Temp, $^\circ$C | Wt. % Adsorbed* |
|---|---|---|---|---|
| $O_2$ | 3.46 | 133 (100) | -183 | 7 |
| $O_2$ | 3.46 | 1000 (750) | -183 | 10 |
| Neopentane | 6.2 | 933 (700) | 24 | 4 |
| $H_2O$ | 2.65 | 5.7 (4.3) | 24 | 4 |
| $H_2O$ | 2.65 | 27 (20.0) | 24 | 12 |

*typical amount adsorbed

The pore diameter of CAPO-5 is greater than 0,62 nm (6.2 Å).

Example 8 (Preparation of CAPO-31)

a) CAPO-31 is prepared from a reaction mixture having a composition, expressed in terms of the molar oxide ratios of the components of the reaction mixture, of:

1.0-2.0 DPA : 0.05-0.2 $Cr_2O_q$ : 0.5-1.0 $Al_2O_3$ : 0.5-1.0 $P_2O_5$ : 40-100 $H_2O$

where "DPA" denotes di-n-propylamine and "q" denotes the oxidation state of chromium.

The reaction mixture is seeded with crystals of $AlPO_4$-31 (U.S. Patent No. 4,310,440) and digested by placing the reaction mixture in a sealed stainless steel pressure vessel and heating it at an effective temperature and for an effective time until crystals of the CAPO-31 product are obtained. Solids are then recovered by filtration, washed with water and dried in air at room temperature.

The CAPO-31 product's chemical analysis shows the CAPO-31 product contains chromium, aluminum and phosphorus in amounts within the hexagonal compositional area defined by points a, b, c, d, e and f of FIG.2.

The x-ray powder diffraction pattern of a CAPO-31 product is characterized by the following data:

| $2\theta$ | $d(nm) \bullet 10^{-1}$ | Relative Intensity |
|---|---|---|
| 8.5 - 8.6 | 10.40 - 10.28 | m - s |
| 20.2 - 20.3 | 4.40 - 4.37 | m |
| 21.9 - 22.1 | 4.06 - 4.02 | w - m |
| 22.6 - 22.7 | 3.93 - 3.92 | vs |
| 31.7 - 31.8 | 2.823 - 2.814 | w - m |

b) The x-ray powder diffraction pattern for a calcined CAPO-31 is also characterized by the X-ray pattern of part a).

c) When the calcined CAPO-31 of part (b) is utilized in adsorption capacity studies using a standard McBain-Bakr gravimetric adsorption apparatus the measurements are made on a sample after activation at $350^\circ$C in a vacuum. The following data are used in the adsorption studies:

| Adsorbate | Kinetic Diameter $(nm) \bullet 10^{-1}$ | Pressure (mbar) (Torr) | Temp, $^\circ$C | Wt. % Adsorbed* |
|---|---|---|---|---|
| $O_2$ | 3.46 | 133 (100) | -183 | 4 |
| $O_2$ | 3.46 | 1000 (750) | -183 | 6 |
| Cyclohexane | 6.0 | 120 (90) | 24 | 3 |
| Neopentane | 6.2 | 933 (700) | 24 | 3 |
| $H_2O$ | 2.65 | 5.7 (4.3) | 24 | 3 |
| $H_2O$ | 2.65 | 27 (20) | 24 | 10 |

* typical amount adsorbed

The pore diameter of CAPO-31 is greater than about 0,6 nm (6.2 Å).

Example 9 (Preparation of GaAPO-5)

a) GaAPO-5 is prepared from a reaction mixture having a composition, expressed in terms of the molar oxide ratios of the components of the reaction mixture, of:

1.0-2.0 TPA : 0.05-0.2 $Ga_2O_3$ : 0.5-1.0 $Al_2O_3$ : 0.5-1.0 $P_2O_5$ : 40-100 $H_2O$

where "TPA" denotes tripropylamine.

The reaction mixture is digested by placing the reaction mixture in a sealed stainless steel pressure vessel and heating it at an effective temperature and for an effective time until crystals of the GaAPO-5 product are obtained. Solids are recovered by filtration, washed with water and dried in air at room temperature.

The GaAPO-5 product's chemical analysis shows the GaAPO-5 product contains gallium, aluminum and phosphorus in amounts within the hexagonal compositional area defined by points a, b, c, d, e and f of FIG.2.

The x-ray powder diffraction pattern of a GaAPO-5 product is characterized by the following data:

| $2\theta$ | $d(nm)\cdot 10^{-1}$ | Relative Intensity |
|---|---|---|
| 7.3 - 7.65 | 12.1 - 11.56 | m - vs |
| 19.5 - 19.95 | 4.55 - 4.46 | m - s |
| 20.9 - 21.3 | 4.25 - 4.17 | m - vs |
| 22.2 - 22.6 | 4.00 - 3.93 | w - vs |
| 25.7 - 26.15 | 3.47 - 3.40 | w - m |

b) The x-ray powder diffraction pattern for a calcined GaAPO-5 is also characterized by the X-ray pattern of part a).

c) When the calcined GaAPO-5 of part (b) is utilized in adsorption capacity studies using a standard McBain-Bakr gravimetric adsorption apparatus the measurements are made on a sample after activation at 350°C in vacuum. The following data are used in the adsorption studies:

| Adsorbate | Kinetic Diameter $(nm)\cdot 10^{-1}$ | Pressure (mbar) (Torr) | Temp, °C | Wt. % Adsorbed* |
|---|---|---|---|---|
| $O_2$ | 3.46 | 133 (100) | -183 | 7 |
| $O_2$ | 3.46 | 1000 (750) | -183 | 10 |
| Neopentane | 6.2 | 933 (700) | 24 | 4 |
| $H_2O$ | 2.65 | 5.7 (4.3) | 24 | 4 |
| $H_2O$ | 2.65 | 27 (20.0) | 24 | 12 |

*typical amount adsorbed

The pore diameter of GaAPO-5 is greater than 0,62 nm (6.2 Å).

Example 10 (Preparation of GaAPO-44)

a) GaAPO-44 is prepared from a reaction mixture having a composition, expressed in terms of the molar oxide ratios of the components of the reaction mixture, of:

1.0-2.0 CHA : 0.05-0.2 $Ga_2O_3$ : 0.5-1.0 $Al_2O_3$ : 0.5-1.0 $P_2O_5$ : 40-100 $H_2O$

where "CHA" denotes cyclohexylamine.

The reaction mixture is digested by placing the reaction mixture in a sealed stainless steel pressure vessel and heating it at an effective temperature and for an effective time until crystals of the GaAPO-44 product are obtained. Solids are then recovered by filtration, washed with water and dried in air at room temperature.

The GaAPO-44 product's chemical analysis shows the GaAPO-44 product contains gallium, aluminum and phosphorus in amounts within the hexagonal compositional area defined by points a, b, c, d, e and f of FIG.2.

The x-ray powder diffraction pattern of a GaAPO-44 product is characterized by the following data:

| $2\theta$ | $d(nm)\cdot 10^{-1}$ | Relative Intensity |
|---|---|---|
| 9.4 - 9.55 | 9.41 - 9.26 | vs |
| 13.0 - 13.1 | 6.81 - 6.76 | w - m |
| 16.0 - 16.2 | 5.54 - 5.47 | w - m |
| 20.6 - 20.85 | 4.31 - 4.26 | s - vs |
| 24.3 - 24.4 | 3.66 - 3.65 | w - vs |
| 30.7 - 30.95 | 2.912 - 2.889 | w - s |

b) When the calcined GaAPO-44 is utilized in adsorption capacity studies using a standard McBain-Bakr gravimetric adsorption apparatus the measurements are made on a sample after activation at 350°C in vacuum. The following data are used in the adsorption studies:

| Adsorbate | Kinetic Diameter (nm)•$10^{-1}$ | Pressure Wt. % (mbar)(Torr) Temp, °C Adsorbed* | |
|---|---|---|---|
| $O_2$ | 3.46 | 133 (100) | -183 13 |
| $O_2$ | 3.46 | 1000 (750) | -183 16 |
| n-hexane | 4.3 | 133 (100) | 24 2 |
| $H_2O$ | 2.65 | 5.7 (4.3) | 24 15 |
| $H_2O$ | 2.65 | 27 (20) | 24 17 |

*typical amount adsorbed

The pore diameter of GaAPO-44 is about 0,43 nm (4.3Å).

Example 11 (Preparation of GeAPO-5)

a) GeAPO-5 is prepared from a reaction mixture having a composition, expressed in terms of the molar oxide ratios of the components of the reaction mixture, of:

1.0-2.0 TPA : 0.1-0.4 $GeO_2$ : 0.5-1.0 $Al_2O_3$ : 0.5-1.0 $P_2O_5$ : 40-100 $H_2O$

where "TPA" denotes tripropylamine.

The reaction mixture is digested by placing the reaction mixture in a sealed stainless steel pressure vessel and heating it at an effective temperature and for an effective time until crystals of the GeAPO-5 product are obtained. Solids are recovered by filtration, washed with water and dried in air at room temperature.

The GeAPO-5 product's chemical analysis shows the GeAPO-5 product contains germanium, aluminum and phosphorus in amounts within the pentagonal compositional area defined by points A, B, C, D, and E of FIG. 1.

The x-ray powder diffraction pattern of a GeAPO-5 product is characterized by the following data:

| $2\theta$ | d(nm)•$10^{-1}$ | Relative Intensity |
|---|---|---|
| 7.3 - 7.65 | 12.1 - 11.56 | m - vs |
| 19.5 - 19.95 | 4.55 - 4.46 | m - s |
| 20.9 - 21.3 | 4.25 - 4.17 | m - vs |
| 22.2 - 22.6 | 4.00 - 3.93 | w - vs |
| 25.7 - 26.15 | 3.47 - 3.40 | w - m |

b) The x-ray powder diffraction pattern for a calcined GeAPO-5 is also characterized by the X-ray pattern of part a).

c) When the calcined GeAPO-5 of part (b) is utilized in adsorption capacity studies using a standard McBain-Bakr gravimetric adsorption apparatus the measurements are made on a sample after activation at 350°C in vacuum. The following data are used in the adsorption studies:

| Adsorbate | Kinetic Diameter (nm)•$10^{-1}$ | Pressure (mbar) (Torr) | Temp, °C | Wt. % Adsorbed* |
|---|---|---|---|---|
| $O_2$ | 3.46 | 133 (100) | -183 | 7 |
| $O_2$ | 3.46 | 1000 (750) | -183 | 10 |
| Neopentane | 6.2 | 933 (700) | 24 | 4 |
| $H_2O$ | 2.65 | 5.7 (4.3) | 24 | 4 |
| $H_2O$ | 2.65 | 27 (20.0) | 24 | 12 |

*typical amount adsorbed

The pore diameter of GeAPO-5 is greater than 0,62 nm (6.2 Å).

Example 12 (Preparation of GeAPO-34)

a) GeAPO-34 is prepared from a reaction mixture having a composition, expressed in terms of the molar oxide ratios of the components of the reaction mixture, of:

1.0-2.0 TEAOH : 0.1-0.4 $GeO_2$ : 0.5-1.0 $Al_2O_3$ : 0.5-1.0 $P_2O_5$ : 40-100 $H_2O$

where "TEAOH" denotes tetraethylammonium hydroxide.

The reaction mixture is digested by placing the reaction mixture in a sealed stainless steel pressure vessel and heating it at an effective temperature and for an effective time until crystals of the GeAPO-34 product are obtained. The solids are recovered by filtration, washed with water and dried in air at room temperature.

The GeAPO-34 product's chemical analysis shows GeAPO-34 contains germanium, aluminum and phosphorus in amounts within the pentagonal compositional area defined by points A, B, C, D, and E of FIG. 1.

The x-ray powder diffraction pattern of a GeAPO-34 product is characterized by the following data:

| $2\theta$ | $d(nm) \cdot 10^{-1}$ | Relative Intensity |
|---|---|---|
| 9.4 - 9.65 | 9.41 - 9.17 | s - vs |
| 15.9 - 16.2 | 5.57 - 5.47 | vw - m |
| 17.85 - 18.4 | 4.97 - 4.82 | w - s |
| 20.3 - 20.9 | 4.37 - 4.25 | m - vs |
| 24.95 - 25.4 | 3.57 - 3.51 | vw - s |
| 30.3 - 30.8 | 2.95 - 2.90 | w - s |

b) The x-ray powder diffraction pattern for a calcined GeAPO-34 is also characterized by the X-ray pattern of part a).

c) When the calcined GeAPO-34 of part (b) is utilized in adsorption capacity studies using a standard McBain-Bakr gravimetric adsorption apparatus the measurements are made on a sample after activation at 350 ° C in vacuum. The following data are used in the adsorption studies:

| Adsorbate | Kinetic Diameter $(nm) \cdot 10^{-1}$ | Pressure (mbar)(Torr) | Temp, ° C | Wt. % Adsorbed* |
|---|---|---|---|---|
| $O_2$ | 3.46 | 133 (100) | -183 | 13 |
| $O_2$ | 3.46 | 1000 (750) | -183 | 18 |
| n-hexane | 4.3 | 133 (100) | 24 | 6 |
| $H_2O$ | 2.65 | 5.7 (4.3) | 24 | 15 |
| $H_2O$ | 2.65 | 27 (20) | 24 | 21 |

*typical amount adsorbed

The pore diameter of GeAPO-34 is about 0,43 nm (4.3Å).

Example 13 (Preparation of LiAPO-5)

a) LiAPO-5 is prepared from a reaction mixture having a composition, expressed in terms of the molar oxide ratios of the components of the reaction mixture, of:

1.0-2.0 TPA : 0.05-0.2 $Li_2O$ : 0.5-1.0 $Al_2O_3$ : 0.5-1.0 $P_2O_5$ : 40-100 $H_2O$

where "TPA" denotes tripropylamine.

The reaction mixture is digested by placing the reaction mixture in a sealed stainless steel pressure vessel and heating it at an effective temperature and for an effective time until crystals of LiAPO-5 product are obtained. Solids are recovered by filtration, washed with water and dried in air at room temperature.

The LiAPO-5 product's chemical analysis shows the LiAPO-5 product contains lithium, aluminum and phosphorus in amounts within the hexagonal compositional area defined by points a, b, c, d, e and f of FIG.2.

The x-ray powder diffraction pattern of a LiAPO-5 product is characterized by the following data:

22

| $2\theta$ | $d(nm) \cdot 10^{-1}$ | Relative Intensity |
|---|---|---|
| 7.3 - 7.65 | 12.1 - 11.56 | m - vs |
| 19.5 - 19.95 | 4.55 - 4.46 | m - s |
| 20.9 - 21.3 | 4.25 - 4.17 | m - vs |
| 22.2 - 22.6 | 4.00 - 3.93 | w - vs |
| 25.7 - 26.15 | 3.47 - 3.40 | w - m |

b) The x-ray powder diffraction pattern for a calcined LiAPO-5 is also characterized by the x-ray pattern of part a).

c) When the calcined LiAPO-5 of part (b) is utilized in adsorption capacity studies using a standard McBain-Bakr gravimetric adsorption apparatus the measurements are made on a sample after activation at 350°C in vacuum. The following data are used in the adsorption studies:

| Adsorbate | Kinetic Diameter $(nm) \cdot 10^{-1}$ | Pressure (mbar)(Torr) | Temp, °C | Wt. %. Adsorbed* |
|---|---|---|---|---|
| $O_2$ | 3.46 | 133 (100) | -183 | 7 |
| $O_2$ | 3.46 | 1000 (750) | -183 | 10 |
| Neopentane | 6.2 | 933 (700) | 24 | 4 |
| $H_2O$ | 2.65 | 5.7 (4.3) | 24 | 4 |
| $H_2O$ | 2.65 | 27 (20.0) | 24 | 12 |

*typical amount adsorbed

The pore diameter of LiAPO-5 is greater than 0,62 nm (6.2 Å).

Example 14 (Preparation of LiAPO-11)

a) LiAPO-11 is prepared from a reaction mixture having a composition, expressed in terms of the molar oxide ratios of the components of the reaction mixture, of:

1.0-2.0 DPA : 0.05-0.2 $Li_2O$ : 0.5-1.0 $Al_2O_3$ : 0.5-1.0 $P_2O_5$ : 40-100 $H_2O$

where "DPA" denotes di-n-propylamine.

The reaction mixture is digested by placing the reaction mixture in a sealed stainless steel pressure vessel and heating it at an effective temperature and for an effective time until crystals of the LiAPO-11 product are obtained. Solids are then recovered by filtration, washed with water and dried in air at room temperature.

The LiAPO-11 product's chemical analysis shows the LiAPO-11 product contains lithium, aluminum and phosphorus in amounts within the hexagonal compositional area defined by points a, b, c, d, e and f of FIG.2.

The x-ray powder diffraction pattern of a LiAPO-11 product is characterized by the following data:

| $2\theta$ | $d(nm) \cdot 10^{-1}$ | Relative Intensity |
|---|---|---|
| 9.3 - 9.65 | 9.51 - 9.17 | m - s |
| 20.2 - 20.6 | 4.40 - 4.31 | m - s |
| 20.9 - 21.3 | 4.25 - 4.17 | s - vs |
| 22.0 - 22.5 | 4.04 - 3.95 | m - s |
| 22.5 - 22.9 | 3.95 - 3.92 | m - s |
| 23.0 - 23.4 | 3.87 - 3.80 | m - vs |

b) The x-ray powder diffraction pattern for a calcined LiAPO-11 is also characterized by the x-ray pattern of part a).

c) When the calcined LiAPO-11 of part (b) is utilized in adsorption capacity studies using a standard McBain-Bakr gravimetric adsorption apparatus the measurements are made on a sample after activation at 350°C in vacuum. The following data are used in the adsorption studies:

| Adsorbate | Kinetic Diameter $(nm) \cdot 10^{-1}$ | Pressure (mbar)(Torr) | Temp, $^{\circ}$C | Wt. % Adsorbed* |
|---|---|---|---|---|
| $O_2$ | 3.46 | 133 (100) | -183 | 5 |
| $O_2$ | 3.46 | 1000 (750) | -183 | 6 |
| Cyclohexane | 6.0 | 120 (90) | 24 | 4 |
| $H_2O$ | 2.65 | 5.7 (4.3) | 24 | 6 |
| $H_2O$ | 2.65 | 27 (20) | 24 | 8 |

*typical amount adsorbed

The pore diameter of LiAPO-11 is about 0,6 nm (6Å).

Example 15 (Preparation of VAPO-5)

a) VAPO-5 is prepared from a reaction mixture having a composition, expressed in terms of the molar oxide ratios of the components of the reaction mixture, of:

1.0-2.0 TPA : 0.05-0.2 $V_2O_q$ : 0.5-1.0 $Al_2O_3$ : 0.5-1.0 $P_2O_5$ : 40-100 $H_2O$

where "TPA" denotes tripropylamine and "q" denotes the oxidation state of vanadium.

The reaction mixture is digested by placing the reaction mixture in a sealed stainless steel pressure vessel and heating it at an effective temperature and for an effective time until crystals of the VAPO-5 product are obtained. Solids are recovered by filtration, washed with water and dried in air at room temperature.

The VAPO-5 product's chemical analysis shows the VAPO-5 product contains vanadium, aluminum and phosphorus in amounts within the hexagonal compositional area defined by points a, b, c, d, e and f of FIG.2.

The x-ray powder diffraction pattern of a VAPO-5 product is characterized by the following data:

| $2\theta$ | $d(nm) \cdot 10^{-1}$ | Relative Intensity |
|---|---|---|
| 7.3- 7.65 | 12.1 - 11.56 | m - vs |
| 19.5-19.95 | 4.55 - 4.46 | m - vs |
| 20.9-21.3 | 4.25 - 4.17 | m - vs |
| 22.2-22.6 | 4.00 - 3.93 | w - vs |
| 25.7-26.15 | 3.47 - 3.40 | w - m |

b) The x-ray powder diffraction pattern for a calcined VAPO-5 is also characterized by the X-ray pattern of part a).

c) When the calcined VAPO-5 of part (b) is utilized in adsorption capacity studies using a standard McBain-Bakr gravimetric adsorption apparatus the measurements are made on a sample after activation at 350$^{\circ}$C in vacuum. The following data are used in the adsorption studies:

| Adsorbate | Kinetic Diameter $(nm) \cdot 10^{-1}$ | Pressure (mbar)(Torr) | Temp, $^{\circ}$C | Wt. % Adsorbed* |
|---|---|---|---|---|
| $O_2$ | 3.46 | 133 (100) | -183 | 7 |
| $O_2$ | 3.46 | 1000 (750) | -183 | 10 |
| Neopentane | 6.2 | 933 (700) | 24 | 4 |
| $H_2O$ | 2.65 | 5.7 (4.3) | 24 | 4 |
| $H_2O$ | 2.65 | 27 (20.0) | 24 | 12 |

*typical amount adsorbed

The pore diameter of VAPO-5 is greater than 0,62 nm (6.2 Å).

Example 16 (Preparation of VAPO-11)

a) VAPO-11 is prepared from a reaction mixture having a composition, expressed in terms of the molar oxide ratios of the components of the reaction mixture, of:

1.0-2.0 DPA : 0.05-0.2 $V_2O_q$ : 0.5-1.0 $Al_2O_3$ : 0.5-1 $P_2O_5$ : 40-100 $H_2O$

where "DPA" denotes di-n-propylamine and "q" denotes the oxidation state of vanadium.

The reaction mixture is digested by placing the reaction mixture in a sealed stainless steel pressure vessel and heating it at an effective temperature and for an effective time until the crystals of the VAPO-11 product are obtained. Solids are then recovered by filtration, washed with water and dried in air at room temperature.

The VAPO-11 product's chemical analysis shows the VAPO-11 product contains vanadium, aluminum and phosphorus in amounts within hexagonal compositional area defined by points a, b, c, d, e and f of FIG.2.

The x-ray powder diffraction pattern of a VAPO-11 product is characterized by the following data:

| $2\theta$ | $d(nm) \cdot 10^{-1}$ | Relative Intensity |
|---|---|---|
| 9.3 - 9.65 | 9.51 - 9.17 | m - s |
| 20.2 - 20.6 | 4.40 - 4.31 | m - s |
| 20.9 - 21.3 | 4.25 - 4.17 | s - vs |
| 22.0 - 22.5 | 4.04 - 3.95 | m - s |
| 22.5 - 22.9 | 3.95 - 3.92 | m - s |
| 23.0 - 23.4 | 3.87 - 3.80 | m - vs |

b) The x-ray powder diffraction pattern for a calcined VAPO-11 is also characterized by the X-ray pattern of part a).

c) When the calcined VAPO-11 of part (b) is utilized in adsorption capacity studies using a standard McBain-Bakr gravimetric adsorption apparatus the measurements are made on a sample after activation at 350°C in vacuum. The following data are used in the adsorption studies:

| Adsorbate | Kinetic Diameter $(nm) \cdot 10^{-1}$ | Pressure (mbar)(Torr) | Temp, °C | Wt. % Adsorbed* |
|---|---|---|---|---|
| $O_2$ | 3.46 | 133 (100) | -183 | 5 |
| $O_2$ | 3.46 | 1000 (750) | -183 | 6 |
| Cyclohexane | 6.0 | 120 (90) | 24 | 4 |
| $H_2O$ | 2.65 | 5.7 (4.3) | 24 | 6 |
| $H_2O$ | 2.65 | 27 (20) | 24 | 8 |

*typical amount adsorbed

The pore diameter of VAPO-11 is about 0.6 nm (6Å).

TABLE V (FCAPO-47)

| $2\theta$ | $d(nm) \cdot 10^{-1}$ | Relative Intensity |
|---|---|---|
| 9.4 | 9.41 | vs |
| 15.9 - 16.0 | 5.57 - 5.54 | w - m |
| 20.5 - 20.6 | 4.33 - 4.31 | s |
| 24.5 - 24.7 | 3.63 - 3.60 | w |
| 25.8 - 25.9 | 3.45 - 3.44 | w |
| 30.4 - 30.5 | 2.940 - 2.931 | w |

Example 17 (Preparation of AsGaAPO-5)

a) AsGaAPO-5 is prepared from a reaction mixture having a composition, expressed in terms of the molar oxide ratios of the components of the reaction mixture, of:

1.0-2.0 TPA : 0.05-0.2 $(M)_2O_q$ : 0.5-1.0 $Al_2O_3$ : 0.5-1.0 $P_2O_5$ : 40-100 $H_2O$

where "TPA" denotes tripropylamine and "q" denotes the oxidation state(s) of "M" (arsenic and gallium).

The reaction mixture is digested by placing the reaction mixture in a sealed stainless steel pressure vessel and heating it at an effective temperature and for an effective time to produce the AsGaAPO-5 product. Solids are recovered by filtration, washed with water and dried in air at room temperature.

The AsGaAPO-5 product's chemical analysis shows the AsGaAPO-5 product contains arsenic, gallium, aluminum and phosphorus in amounts within the pentagonal compositional area defined by points A, B, C, D and E of FIG. 1.

The x-ray powder diffraction pattern of a AsGaAPO-5 product is characterized by the following data:

| $2\theta$ | $d(nm) \cdot 10^{-1}$ | Relative Intensity |
|---|---|---|
| 7.3 - 7.65 | 12.1 - 11.56 | m - vs |
| 19.5 - 19.95 | 4.55 - 4.46 | m - s |
| 20.9 - 21.3 | 4.25 - 4.17 | m - vs |
| 22.2 - 22.6 | 4.00 - 3.93 | w - vs |
| 25.7 - 26.15 | 3.47 - 3.40 | w - m |

b) The x-ray powder diffraction pattern for a calcined AsGaAPO-5 is also characterized by the pattern of part a).

c) When the calcined AsGaAPO-5 of part (b) is utilized in adsorption capacity studies using a standard McBain-Bakr gravimetric adsorption apparatus the measurements are made on a sample after activation at 350 $^\circ$C in a vacuum. The following data are used in the adsorption studies:

| Adsorbate | Kinetic Diameter $(nm) \cdot 10^{-1}$ | Pressure (mbar)(Torr) | Temp, $^\circ$C | Wt. % Adsorbed* |
|---|---|---|---|---|
| $O_2$ | 3.46 | 133 (100) | -183 | 7 |
| $O_2$ | 3.46 | 1000 (750) | -183 | 10 |
| Neopentane | 6.2 | 933 (700) | 24 | 4 |
| $H_2O$ | 2.65 | 5.7 (4.3) | 24 | 4 |
| $H_2O$ | 2.65 | 27 (20.0) | 24 | 12 |

*typical amount adsorbed

The pore diameter of AsGaAPO-5 is greater than 0,62 nm (6.2 Å).

Example 18 (Preparation of BeGeAPO-11)

a) BeGeAPO-11 is prepared from a reaction mixture having a composition, expressed in terms of the molar oxide ratios of the components of the reaction mixture, of:

1.0-2.0 DPA : 0.05-0.2 $(M)_2O_q$ : 0.5-1.0 $Al_2O_3$ : 0.5-1.0 $P_2O_5$ : 40-100 $H_2O$

where "DPA" denotes di-n-propylamine and "q" denotes the oxidation state(s) of "M" (beryllium and germanium).

The reaction mixture is digested by placing the reaction mixture in a sealed stainless steel pressure vessel and heating it at an effective temperature and for an effective time to produce the BeGeAPO-11 product. Solids are then recovered by filtration, washed with water and dried in air at room temperature.

The BeGeAPO-11 product's chemical analysis shows the BeGeAPO-11 product contains beryllium, germanium, aluminum and phosphorus in amounts within the pentagonal compositional area defined by points A, B, C, D and E of FIG. 1.

The x-ray powder diffraction pattern of a BeGeAPO-11 product is characterized by the following data:

| $2\theta$ | $d(nm) \cdot 10^{-1}$ | Relative Intensity |
|---|---|---|
| 9.3 - 9.65 | 9.51 - 9.17 | m - s |
| 20.2 - 20.6 | 4.40 - 4.31 | m - s |
| 20.9 - 21.3 | 4.25 - 4.17 | s - vs |
| 22.0 - 22.5 | 4.04 - 3.95 | m - s |
| 22.5 - 22.9 | 3.95 - 3.92 | m - s |
| 23.0 - 23.4 | 3.87 - 3.80 | m - vs |

b) The x-ray powder diffraction pattern for a calcined BeGeAPO-11 is also characterized by the X-ray pattern of part a).

c) When the calcined BeGeAPO-11 of part (b) is utilized in adsorption capacity studies using a standard McBain-Bakr gravimetric adsorption apparatus the measurements are made on a sample after activation at 350°C in a vacuum. The following data are used in the adsorption studies:

| Adsorbate | Kinetic Diameter $(nm) \cdot 10^{-1}$ | Pressure (mbar)(Torr) | Temp, °C | Wt. % Adsorbed* |
|---|---|---|---|---|
| $O_2$ | 3.46 | 133 (100) | -183 | 5 |
| $O_2$ | 3.46 | 1000 (750) | -183 | 6 |
| Cyclohexane | 6.0 | 120 (90) | 24 | 4 |
| $H_2O$ | 2.65 | 5.7 (4.3) | 24 | 6 |
| $H_2O$ | 2.65 | 27 (20) | 24 | 8 |

*typical amount adsorbed

The pore diameter of BeGeAPO-11 is about 0,6 nm (6Å).

Example 19 (Preparation of CrGaAPO-17)

a) CrGaAPO-17 is prepared from a reaction mixture having a composition, expressed in terms of the molar oxide ratios of the components of the reaction mixture, of:

1.0-2.0 QN : 0.05-0.2 $(M)_2O_q$ : 0.5-1.0 $Al_2O_3$ : 0.5-1.0 $P_2O_5$: 40-100 $H_2O$

where "QN" denotes quinuclidine and "q" denotes the oxidation state(s) of "M" (chromium and gallium).

The reaction mixture is digested by placing the reaction mixture in a sealed stainless steel pressure vessel and heating it at an effective temperature and for an effective time to produce the CrGaAPO-17 product. Solids are then recovered by filtration, washed with water and dried in air at room temperature.

The CrGaAPO-17 product's chemical analysis shows the CrGaAPO-17 product contains chromium, gallium, aluminum and phosphorus in amounts within the pentagonal compositional area defined by points A, B, C, D and E of FIG. 1.

The x-ray powder diffraction pattern of a CrGaAPO-17 product is characterized by the following data:

| $2\theta$ | $d(nm) \cdot 10^{-1}$ | Relative Intensity |
|---|---|---|
| 7.7 - 7.75 | 11.5 - 11.4 | vs |
| 13.4 | 6.61 | s - vs |
| 15.5 - 15.55 | 5.72 - 5.70 | s |
| 19.65 - 19.7 | 4.52 - 4.51 | w - s |
| 20.5 - 20.6 | 4.33 - 4.31 | vs |
| 31.8 - 32.00 | 2.812 - 2.797 | w - s |

b) The x-ray powder diffraction pattern for a calcined CrGaAPO-17 is also characterized by the X-ray pattern of part a).

c) When the calcined CrGaAPO-17 of part (b) is utilized in adsorption capacity studies using a standard McBain-Bakr gravimetric adsorption apparatus the measurements are made on a sample after activation at 350°C in a vacuum. The following data are used in the adsorption studies:

| Adsorbate | Kinetic Diameter $(nm) \cdot 10^{-1}$ | Pressure (mbar)(Torr) | Temp, $^\circ$C | Wt. % Adsorbed* |
|---|---|---|---|---|
| $O_2$ | 3.46 | 133 (100) | -183 | 10 |
| $O_2$ | 3.46 | 1000 (750) | -183 | 12 |
| n-butane | 4.3 | 133 (100) | 24 | 4 |
| $H_2O$ | 2.65 | 5.7 (4.3) | 24 | 13 |
| $H_2O$ | 2.65 | 27 (20) | 24 | 14 |

*typical amount adsorbed

The pore diameter of CrGaAPO-17 is about 0,43 nm (4.3 Å).

Example 20 (Preparation of AsBeAPO-31)

a) AsBeAPO-31 is prepared from a reaction mixture having a composition, expressed in terms of the molar oxide ratios of the components of the reaction mixture, of:

1.0-2.0 DPA : 0.05-0.2 $(M)_2O_q$ : 0.5-1.0 $Al_2O_3$ : 0.5-1.0 $P_2O_5$ : 40-100 $H_2O$

where "DPA" denotes di-n-propylamine and "q" denotes the oxidation state of "M" (arsenic and beryllium).

The reaction mixture is seeded with crystals of $AlPO_4$-31 (U.S. Patent No. 4,310,440) and digested by placing the reaction mixture in a sealed stainless steel pressure vessel and heating it at an effective temperature and for an effective time to produce the AsBeAPO-31 product. Solids are then recovered by filtration, washed with water and dried in air at room temperature.

The AsBeAPO-31 product's chemical analysis shows the AsBeAPO-31 product contains arsenic, beryllium, aluminum and phosphorus in amounts within the pentagonal compositional area defined by points A, B, C, D and E of FIG. 1.

The x-ray powder diffraction pattern of a AsBeAPO-31 product is characterized by the following data:

| $2\theta$ | $d(nm) \cdot 10^{-1}$ | Relative Intensity |
|---|---|---|
| 8.5 - 8.6 | 10.40 - 10.28 | m - s |
| 20.2 - 20.3 | 4.40 - 4.37 | m |
| 21.9 - 22.1 | 4.06 - 4.02 | w - m |
| 22.6 - 22.7 | 3.93 - 3.92 | vs |
| 31.7 - 31.8 | 2.823 - 2.814 | w - m |

b) The x-ray powder diffraction pattern for a calcined AsBeAPO-31 is also characterized by the X-ray pattern of part a).

c) When the calcined AsBeAPO-31 of part (b) is utilized in adsorption capacity studies using a standard McBain-Bakr gravimetric adsorption apparatus the measurements are made on a sample after activation at 350$^\circ$C in a vacuum. The following data are used in the adsorption studies:

| Adsorbate | Kinetic Diameter $(nm) \cdot 10^{-1}$ | Pressure (mbar)(Torr) | Temp, $^\circ$C | Wt. % Adsorbed* |
|---|---|---|---|---|
| $O_2$ | 3.46 | 133 (100) | -183 | 4 |
| $O_2$ | 3.46 | 1000 (750) | -183 | 6 |
| Cyclohexane | 6.0 | 120 (90) | 24 | 3 |
| Neopentane | 6.2 | 933 (700) | 24 | 3 |
| $H_2O$ | 2.65 | 5.7 (4.3) | 24 | 3 |
| $H_2O$ | 2.65 | 27 (20) | 24 | 10 |

*typical amount adsorbed

The pore diameter of AsBeAPO-31 is greater than about 0,62 nm (6.2 Å).

Example 21 (Preparation of AsVBeAPO-34)

28

a) AsVBeAPO-34 is prepared from a reaction mixture having a composition, expressed in terms of the molar oxide ratios of the components of the reaction mixture, of:

1.0-2.0 TEAOH : 0.05-0.2 $(M)_2O_q$ : 0.5-1.0 $Al_2O_3$ : 0.5-1.0 $P_2O_5$ : 40-100 $H_2O$

where "TEAOH" denotes tetraethylammonium hydroxide and "q" denotes the oxidation state of "M" (arsenic, vanadium, and beryllium).

The reaction mixture is digested by placing the reaction mixture in a sealed stainless steel pressure vessel and heating it at an effective temperature and for an effective time to produce the AsVBeAPO-34 product. The solids are recovered by filtration, washed with water and dried in air at room temperature.

The AsVBeAPO-34 product's chemical analysis shows the AsVBeAPO-34 product contains arsenic, vanadium, beryllium, aluminum and phosphorus in amounts within the pentagonal compositional area defined by points A, B, C, D and E of FIG. 1.

The x-ray powder diffraction pattern of a AsVBeAPO-34 product is characterized by the following data:

| $2\theta$ | $d(nm) \cdot 10^{-1}$ | Relative Intensity |
|---|---|---|
| 9.4 - 9.65 | 9.41 - 9.17 | s - vs |
| 15.9 - 16.2 | 5.57 - 5.47 | vw - m |
| 17.85 - 18.4 | 4.97 - 4.82 | w - s |
| 20.3 - 20.9 | 4.37 - 4.25 | m - vs |
| 24.95 - 25.4 | 3.57 - 3.51 | vw - s |
| 30.3 - 30.8 | 2.95 - 2.90 | w - s |

b) The x-ray powder diffraction pattern for a calcined AsVBeAPO-34 is pattern of part a).

c) When the calcined AsVBeAPO-34 of part (b) is utilized in adsorption capacity studies using a standard McBain-Bakr gravimetric adsorption apparatus the measurements are made on a sample after activation at 350°C in a vacuum. The following data are used in the adsorption studies:

| Adsorbate | Kinetic Diameter $(nm) \cdot 10^{-1}$ | Pressure (mbar)(Torr) | Temp, °C | Wt. % Adsorbed* |
|---|---|---|---|---|
| $O_2$ | 3.46 | 133 (100) | -183 | 13 |
| $O_2$ | 3.46 | 1000 (750) | -183 | 18 |
| n-hexane | 4.3 | 133 (100) | 24 | 6 |
| $H_2O$ | 2.65 | 5.7 (4.3) | 24 | 15 |
| $H_2O$ | 2.65 | 27 (20) | 24 | 21 |

*typical amount adsorbed

The pore diameter of AsVBeAPO-34 is about 0,43 nm (4.3Å).

Example 22 (Preparation of GeBAPO-44)

a) GeBAPO-44 is prepared from a reaction mixture having a composition, expressed in terms of the molar oxide ratios of the components of the reaction mixture, of:

1.0-2.0 CHA : 0.05-0.2 $(M)_2O_q$ : 0.5-1.0 $Al_2O_3$ : 0.5-1.0 $P_2O_5$ : 40-100 $H_2O$

where "CHA" denotes cyclohexylamine and "q" denotes the oxidation state of "M" (boron and germanium).

The reaction mixture is digested by placing the reaction mixture in a sealed stainless steel pressure vessel and heating it at an effective temperature and for an effective time to produce GeBAPO-44 product. Solids are then recovered by filtration, washed with water and dried in the air at room temperature.

The GeBAPO-44 product's chemical analysis shows the GeBAPO-44 product contains boron, germanium, aluminum and phosphorus in amounts within the pentagonal compositional area defined by points A, B, C, D and E of FIG. 1.

The x-ray powder diffraction pattern of a GeBAPO-44 product is characterized by the following data:

EP 0 158 976 B1

| 2θ | d(nm)•10⁻¹ | Relative Intensity |
|---|---|---|
| 9.4 - 9.55 | 9.41 - 9.26 | vs |
| 13.0 - 13.1 | 6.81 - 6.76 | w - m |
| 16.0 - 16.2 | 5.54 - 5.47 | w - m |
| 20.6 - 20.85 | 4.31 - 4.26 | s - vs |
| 24.3 - 24.4 | 3.66 - 3.65 | w - vs |
| 30.7 - 30.95 | 2.912 - 2.889 | w - s |

b) When the calcined GeBAPO-44 is utilized in adsorption capacity studies using a standard McBain-Bakr gravimetric adsorption apparatus the measurements are made on a sample after activation at 350°C in a vacuum. The following data are used in the adsorption studies:

| Adsorbate | Kinetic Diameter (nm)•10⁻¹ | Pressure (mbar)(Torr) | Temp, °C | Wt. % Adsorbed* |
|---|---|---|---|---|
| $O_2$ | 3.46 | 133 (100) | -183 | 13 |
| $O_2$ | 3.46 | 1000 (750) | -183 | 16 |
| n-hexane | 4.3 | 133 (100) | 24 | 2 |
| $H_2O$ | 2.65 | 5.7 (4.3) | 24 | 15 |
| $H_2O$ | 2.65 | 27 (20) | 24 | 17 |

*typical amount adsorbed

The pore diameter of GeBAPO-44 is about 0,43 nm (4.3Å).

PROCESS APPLICATIONS

The FCAPO compositions of the present invention are, in general, hydrophilic and adsorb water preferentially over common hydrocarbon molecules such as paraffins, olefins and aromatic species, e.g., benzene, xylenes and cumene. Thus, the present FCAPO compositions as a class are useful as desiccants in such adsorption separation/purification processes as natural gas drying, cracked gas drying. Water is also preferentially adsorbed over the so-called permanent gases such as carbon dioxide, nitrogen, oxygen and hydrogen. These FCAPOs are therefore suitably employed in the drying of reformer hydrogen streams and in the drying of oxygen, nitrogen or air prior to liquifaction.

The present FCAPO compositions also exhibit novel surface selectivity characteristics which render them useful as catalyst or catalyst bases in a number of hydrocarbon conversion and oxidative combustion reactions. They can be impregnated or otherwise loaded with catalytically active metals by methods well known in the art and used, for example, in fabricating catalyst compositions having silica or alumina bases. Of the general class, those species having pores larger than about 0,4 nm (4Å) are preferred for catalytic applications.

Among the hydrocarbon conversion reactions catalyzed by FCAPO compositions are cracking, hydrocracking, alkylation for both the aromatic and isoparaffin types, isomerization including xylene isomerization, polymerization, reforming, hydrogenation, dehydrogenation, transalkylation, dealkylation, hydrodecyclization and dehydrocyclization.

Using FCAPO catalyst compositions which contain a hydrogenation promoter such as platinum or palladium, heavy petroleum residual stocks, cyclic stocks and other hydrocrackable charge stocks, can be hydrocracked at temperatures in the range of 204°C (400°F) to 441.°C (825°F) using molar ratios of hydrogen to hydrocarbon in the range of between 2 and 80, pressures between 1,7 and 242 bar (10 and 3500 p.s.i.g.), and a liquid hourly space velocity (LHSV) of from 0.1 to 20, preferably 1.0 to 10.

The FCAPO catalyst compositions employed in hydrocracking are also suitable for use in reforming processes in which the hydrocarbon feedstocks contact the catalyst at temperatures of from about 371°C (700°F) to 538°C (1000°F), hydrogen pressures of from 7,9 to 35,5 bar (100 to 500 p.s.i.g.), LHSV values in the range of 0.1 to 10 and hydrogen to hydrocarbon molar ratios in the range of 1 to 20, preferably between 4 and 12.

These same catalysts, i.e. those containing hydrogenation promoters, are also useful in hydroisomerizations processes in which feedstocks such as normal paraffins are converted to saturated branched chain isomers. Hydroisomerization is carried out at a temperature of from about 93°C (200°F) to 316°C (600°F),

30

EP 0 158 976 B1

preferably 149°C (300°F) to 288°C (550°F) with an LHSV value of from about 0.2 to 1.0. Hydrogen is supplied to the reactor in admixture with the hydrocarbon feedstock in molar proportions (hydrogen/hydrocarbon) of between 1 and 5.

At somewhat higher temperatures, i.e. from about 343°C (650°F) to 538°C 1000°F), preferably 454°C (850°F) to 510°C (950°F) and usually at somewhat lower pressures within the range of about 2 to 4,5 bar (15 to 50 p.s.i.g.), the same catalyst compositions are used to hydroisomerize normal paraffins. Preferably the paraffin feedstock comprises normal paraffins having a carbon number range of $C_7$-$C_{20}$. Contact time between the feedstock and the catalyst is generally relatively short to avoid undesirable side reactions such as olefin polymerization and paraffin cracking. LHSV values in the range of 0.1 to 10, preferably 1.0 to 6.0 are suitable.

The unique crystal structure of the present FCAPO catalysts and their availability in a form totally void of alkali metal content favor their use in the conversion of alkylaromatic compounds, particularly the catalytic disproportionation of toluene, ethylene, trimethyl benzenes, tetramethyl benzenes and the like. In the disproportionation process, isomerization and transalkylation can also occur. Group VIII noble metal adjuvants alone or in conjunction with Group VI-B metals such as tungsten, molybdenum and chromium are preferably included in the catalyst composition in amounts of from about 3 to 15 weight-% of the overall composition. Extraneous hydrogen can, but need not, be present in the reaction zone which is maintained at a temperature of from about 20,4 to 399°C (400 to 750°F), pressures in the range of 7,9 to 139 bar (100 to 2000 p.s.i.g.) and LHSV values in the range of 0.1 to 15.

Catalytic cracking processes are preferably carried out with FCAPO compositions using feedstocks such as gas oils, heavy naphthas, deasphalted crude oil residua, etc., with gasoline being the principal desired product. Temperature conditions of 454 to 593°C (850 to 1100°F), LHSV values of 0.5 to 10 and pressure conditions of from about 1 to 4,5 bar (0 to 50 p.s.i.g.) are suitable.

Dehydrocyclization reactions employing paraffinic hydrocarbon feedstocks, preferably normal paraffins having more than 6 carbon atoms, to form benzene, xylenes, toluene and the like are carried out using essentially the same reaction conditions as for catalytic cracking. For these reactions it is preferred to use the FCAPO catalyst in conjunction with a Group VIII non-noble metal cation such as cobalt and nickel.

In catalytic dealkylation wherein it is desired to cleave paraffinic side chains from aromatic nuclei without substantially hydrogenating the ring structure, relatively high temperatures in the range of about 427°C - 538°C (800°-1000°F) are employed at moderate hydrogen pressures of about 22-70 bar (300-1000 p.s.i.g.), other conditions being similar to those described above for catalytic hydrocracking. Preferred catalysts are of the same type described above in connection with catalytic dehydrocyclization. Particularly desirable dealkylation reactions contemplated herein include the conversion of methylnaphthalene to naphthalene and toluene and/or xylenes to benzene.

In catalytic hydrofining, the primary objective is to promote the selective hydrodecomposition of organic sulfur and/or nitrogen compounds in the feed, without substantially affecting hydrocarbon molecules therein. For this purpose it is preferred to employ the same general conditions described above for catalytic hydrocracking, and catalysts of the same general nature described in connection with dehydrocyclization operations. Feedstocks include gasoline fractions, kerosenes, jet fuel fractions, diesel fractions, light and heavy gas oils, deasphalted crude oil residua and the like any of which may contain up to about 5 weight-percent of sulfur and up to about 3 weight-percent of nitrogen.

Similar conditions can be employed to effect hydrofining, i.e., denitrogenation and desulfurization, of hydrocarbon feeds containing substantial proportions of organonitrogen and organosulfur compounds. It is generally recognized that the presence of substantial amounts of such constituents markedly inhibits the activity of hydrocracking catalysts. Consequently, it is necessary to operate at more extreme conditions when it is desired to obtain the same degree of hydrocracking conversion per pass on a relatively nitrogenous feed than are required with a feed containing less organonitrogen compounds. Consequently, the conditions under which denitrogenation, desulfurization and/or hydrocracking can be most expeditiously accomplished in any given situation are necessarily determined in view of the characteristics of the feedstocks in particular the concentration of organonitrogen compounds in the feedstock. As a result of the effect of organonitrogen compounds on the hydrocracking activity of these compositions it is not at all unlikely that the conditions most suitable for denitrogenation of a given feedstock having a relatively high organonitrogen content with minimal hydrocracking, e.g., less than 20 volume percent of fresh feed per pass, might be the same as those preferred for hydrocracking another feedstock having a lower concentration of hydrocracking inhibiting constituents e.g., organonitrogen compounds. Consequently, it has become the practice in this art to establish the conditions under which a certain feed is to be contacted on the basis of preliminary screening tests with the specific catalyst and feedstock.

Isomerization reactions are carried out under conditions similar to those described above for reforming,

31

using somewhat more acidic catalysts. Olefins are preferably isomerized at temperatures of 260°C (500°)-482°C (900°F), while paraffins, naphthenes and alkyl aromatics are isomerized at temperatures of 371°C (700°)-538°C (1000°F). Particularly desirable isomerization reactions contemplated herein include the conversion of n-heptene and/or n-octane to isoheptanes, iso-octanes, butane to iso-butane, methylcyclopentane to cyclohexane, meta-xylene and/or ortho-xylene to paraxylene, 1-butene to 2-butene and/or isobutene, n-hexene to isohexene, cyclohexene to methylcyclopentene etc. The preferred form of the catalyst is a combination of the FCAPO with polyvalent metal compounds (such as sulfides) of metals of Group II-A, Group II-B and rare earth metals. For alkylation and dealkylation processes the FCAPO compositions having pores of at least 0,5 nm (5Å) are preferred. When employed for dealkylation of alkyl aromatics, the temperature is usually at least 177°C (350°F) and ranges up to a temperature at which substantial cracking of the feedstock or conversion products occurs, generally up to about 371°C (700°F). The temperature is preferably at least 232°C (450°F) and not greater than the critical temperature of the compound undergoing dealkylation. Pressure conditions are applied to retain at least the aromatic feed in the liquid state. For alkylation the temperature can be as low as 121°C (250°F) but is preferably at least 177°C (350°F). In the alkylation of benzene, toluene and xylene, the preferred alkylating agents are olefins such as ethylene and propylene.

**Claims**

1. Crystalline molecular sieves having three-dimensional microporous framework structures of $MO_2$, $AlO_2$, and $PO_2$ tetrahedral units having an empirical chemical composition on an anhydrous basis expressed by the formula:

   $$mR : (M_xAl_yP_z)O_2$$

   wherein "R" represents at least one organic templating agent present in the intracrystalline pore system; "m" represents the molar amount of "R" present per mole of $(M_xAl_yP_z)O_2$ and has a value of zero to about 0.3; "M" represents at least one element selected from the group consisting of arsenic, beryllium, boron, chromium, gallium, germanium, lithium and vanadium; and "x", "y" and "z" represent the mole fractions of "M", aluminum and phosphorus respectively, present as tetrahedral oxides, said mole fractions being such that they are within the pentagonal compositional area defined by points A, B, C, D and E of FIG. 1.

2. Molecular sieves according to claim 1 wherein the mole fractions of "M", aluminum and phosphorus present as tetrahedral oxides are within the hexagonal compositional area defined by points a, b, c, d, e and f of FIG. 2.

3. The crystalline molecular sieves of claim 1 wherein "M" is arsenic.

4. The crystalline molecular sieves of claim 1 wherein "M" is beryllium.

5. The crystalline molecular sieves of claim 1 wherein "M" is boron.

6. The crystalline molecular sieves of claim 1 wherein "M" is chromium.

7. The crystalline molecular sieves of claim 1 wherein "M" is gallium.

8. The crystalline molecular sieves of claim 1 wherein "M" is lithium.

9. The crystalline molecular sieves of claim 1 wherein "M" is vanadium.

10. The crystalline molecular sieves of claims 1 or 2 having a characteristic x-ray powder diffraction pattern which contains at least the d-spacings set forth in Table A.

11. The crystalline molecular sieves of claims 1 or 2 having a characteristic x-ray powder diffraction pattern which contains at least the d-spacings set forth in Table B.

12. The crystalline molecular sieves of claims 1 or 2 having a characteristic x-ray powder diffraction pattern which contains at least the d-spacings set forth in Table C.

13. The crystalline molecular sieves of claims 1 or 2 having a characteristic x-ray powder diffraction pattern which contains at least the d-spacings set forth in Table D.

14. The crystalline molecular sieves of claims 1 or 2 having a characteristic x-ray powder diffraction pattern which contains at least the d-spacings set forth in Table E.

15. The crystalline molecular sieves of claims 1 or 2 having a characteristic x-ray powder diffraction pattern which contains at least the d-spacings set forth in Table F.

16. The crystalline molecular sieves of claims 1 or 2 having a characteristic x-ray powder diffraction pattern which contains at least the d-spacings set forth in Table G.

17. The crystalline molecular sieves of claims 1 or 2 having a characteristic x-ray powder diffraction pattern which contains at least the d-spacings set forth in Table H.

18. The crystalline molecular sieves of claims 1 or 2 having a characteristic x-ray powder diffraction pattern which contains at least the d-spacings set forth in Table J.

19. The crystalline molecular sieves of claims 1 or 2 having a characteristic x-ray powder diffraction "pattern which contains at least the d-spacings set forth in Table K.

20. The crystalline molecular sieves of claims 1 or 2 having a characteristic x-ray powder diffractions pattern which contains at least the d-spacing set forth in Table L.

21. The crystalline molecular sieves of claims 1 or 2 having a characteristic x-ray powder diffraction pattern which contains at least the d-spacings set forth in Table M.

22. The crystalline molecular sieves of claims 1 or 2 having a characteristic x-ray powder diffraction pattern which contains at least the d-spacings set forth in Table N.

23. The crystalline molecular sieves of claims 1 or 2 having a characteristic x-ray powder diffraction pattern which contains at least the d-spacings set forth in Table O.

24. The crystalline molecular sieves of claims 1 or 2 having a characteristic x-ray powder diffraction pattern which contains at least the d-spacings set forth in Table P.

25. The crystalline molecular sieves of claims 1 or 2 having a characteristic x-ray powder diffraction pattern which contains at least the d-spacings set forth in Table Q.

26. The crystalline molecular sieves of claims 1 or 2 having a characteristic x-ray powder diffraction pattern which contains at least the d-spacings set forth in Table R.

27. The crystalline molecular sieves of claims 1 or 2 having a characteristic x-ray powder diffraction pattern which contains at least the d-spacings set forth in Table S.

28. The crystalline molecular sieves of claims 1 or 2 having a characteristic x-ray powder diffraction pattern which contains at least the d-spacings set forth in Table T.

29. The crystalline molecular sieves of claims 1 or 2 having a characteristic x-ray powder diffraction pattern which contains at least the d-spacings set forth in Table U.

30. The crystalline molecular sieves of claims 1 or 2 having a characteristic x-ray powder diffraction pattern which contains at least the d-spacings set forth in Table V.

31. The crystalline molecular sieves according to claim 1 or 2 wherein at least two elements "M" are selected.

EP 0 158 976 B1

**32.** The molecular sieves according to claim 31 wherein "M" is arsenic and germanium.

**33.** Molecular sieves according to claim 31 wherein "M" is beryllium and germanium.

**34.** Molecular sieves according to claim 31 wherein "M" is chromium and gallium.

**35.** The molecular sieves according to claim 31 wherein "M" is arsenic and beryllium.

**36.** The molecular sieves according to claim 31 wherein "M" is arsenic, vanadium and beryllium.

**37.** The molecular sieves of claim 31 wherein "M" is germanium and boron.

**38.** The molecular sieves of claim 31 wherein "M" is at least two elements selected from the group consisting of boron, chromium, vanadium, arsenic, germanium and gallium.

**39.** The molecular sieves of claim 31 wherein "M" is germanium and at least one element selected from the group consisting of beryllium, boron and gallium.

**40.** Process for preparing the crystalline molecular sieves of claim 1 which comprises providing a reaction mixture composition expressed in terms of molar oxide ratios as follows:

$$aR : (M_xAl_yP_z)O_2 : bH_2O$$

wherein "R" is an organic templating agent: "a" is the amount of "R" and may be zero or an effective amount greater than zero: "b" has a value of from zero to about 500, "M" represents at least two elements capable of forming framework tetrahedral oxides and selected from the group consisting of arsenic, beryllium, boron, chromium, gallium, germanium, lithium and vanadium; "x", "y" and "z" represent the mole fractions, respectively, of "M", aluminum and phosphorus in the $(M_xAl_yP_z)O_2$ constituent, and each has a value of at least 0.01, whereby the molecular sieves of claim 1 are prepared.

**41.** The process of claim 40 where "x", "y" and "z" are within the hexagonal compositional area defined by points F, G, H, I, J and K which is Fig. 3 of the drawings.

**42.** Process according to claim 40 wherein "a" is an effective amount from greater than zero to about 6.

**43.** Process according to claim 40 wherein the source of phosphorus in the reaction mixture is orthophosphoric acid.

**44.** Process according to claim 40 wherein the source of phosphorus in the reaction mixture is orthophosphoric acid and the source of aluminum is at least one compound selected from the group of pseudo-boehmite and aluminum alkoxide.

**45.** Process according to claim 44 wherein the aluminum alkoxide is aluminum isopropoxide.

**46.** Process according to claim 40 wherein the source of "M" is selected from the group consisting of chlorides, bromide, iodides, sulfates, nitrates, acetates and formates.

**47.** Process according to claim 40 wherein the organic templating agent is a quaternary ammonium or quaternary phosphonium compound having the formula:

$$R_4X^+$$

wherein X is nitrogen or phosphorus and each R is an alkyl or aryl group containing from 1 to 8 carbon atoms.

**48.** Process according to claim 40 wherein the organic templating agent is an amine.

34

**49.** Process according to claim 40 wherein the templating agent is selected from the group consisting of tetrapropylammonium ion; tetraethylammonium ion; tripropylamine; triethylamine; triethanolamine; piperidine; cyclohexylamine; 2-methyl pyridine' N,N-dimethylbenzylamine; N,N-diethylethanolamine; choline; N,N-dimethylpiperazine; 1,4-diaziabicyclo-(2,2,2) octane; N-methyldiethanolamine; N-methylethanolamine; N-methylpiperidine; 3-methylpiperidine; N-methylcyclohexylamine; 3-methylpyridine; 4-methylpyridine; quinuclidine; N,N'-dimethyl-1,4-diaziabicyclo (2,2,2) octane ion; tetramethylammonium ion; tetrabutylammonium ion; tetrapentylammonium ion; di-n-butylamine; neopentylamine; di-n-pentylamine; isopropylamine; t-butylamine; ethylenediamine; pyrrolidine; 2-imidazolidone; di-n-propylamine; and a polymeric quaternary ammonium salt $[(C_{14}H_{32}N_2)(OH)_2]_x$ wherein x is a value of at least 2.

**50.** Molecular sieve prepared by calcining the compositions of claim 1 or claim 2 at a temperature sufficiently high to remove at least some of any organic templating agent present in the intracrystalline pore system.

**51.** Process for separating molecular species from admixture with molecular species having a lesser degree of polarity which comprises contacting said mixture of molecular species with a molecular sieve of claim 1 having pore diameter large enough to adsorb at least one of the more polar molecular species, said molecular sieve being at least partially activated whereby molecules of the more polar molecular species are selectively adsorbed into the intracrystalline pore system thereof.

**52.** Process for separating a mixture of molecular species having different kinetic diameters which comprises contacting said mixture with a molecular sieve of claim 1 having pore diameters large enough to adsorb at least one but not all molecular species of said mixture, said molecular sieve being at least partially activated whereby at least some molecules whose kinetic diameters are sufficiently small can enter the intracrystalline pore system thereof.

**53.** Process according to claim 51 wherein the more polar molecular species is water.

**54.** Process for converting a hydrocarbon which comprises contacting said hydrocarbon under hydrocarbon converting conditions with a molecular sieve of claim 1 or claim 2.

**55.** Process according to claim 54 wherein the hydrocarbon conversion process is cracking.

**56.** Process according to claim 54 wherein the hydrocarbon conversion process is hydrocracking.

**57.** Process according to claim 54 wherein the hydrocarbon conversion process is hydrogenation.

**58.** Process according to claim 54 wherein the hydrocarbon conversion process is polymerization.

**59.** Process according to claim 54 wherein the hydrocarbon conversion process is alkylation.

**60.** Process according to claim 54 wherein the hydrocarbon conversion process is reforming.

**61.** Process according to claim 54 wherein the hydrocarbon conversion process is hydrotreating.

**62.** Process according to claim 54 wherein the hydrocarbon conversion process is isomerization.

**63.** Process according to claim 62 wherein the isomerization conversion process is xylene isomerization.

**64.** Process according to claim 54 wherein the hydrocarbon conversion process is dehydrocyclization.

**Revendications**

**1.** Tamis moléculaire cristallin à structures en réseau tridimensionnel microporeux, comprenant des motifs tétraédriques $MO_2$, $AlO_2$, et $PO_2$, ayant une composition chimique empirique exprimée, par rapport à l'état anhydre, par la formule :

mR : $(M_xAl_yP_z)O_2$

dans laquelle "R" représente au moins un agent organique formant matrice présent dans le système de pores intracristallins ; "M" représente la quantité molaire de "R" présente par moles de $(M_xAl_yP_z)O_2$ et a une valeur de 0 à environ 0,3 ; "M" représente au moins un élément choisi parmi l'arsenic, le béryllium, le bore, le chrome, le gallium, le germanium, le lithium et le vanadium ; et "x", "y" et "z" représentent respectivement les fractions molaires de "M", d'aluminium et de phosphore, présents sous la forme d'oxydes tétraédriques, ces fractions étant telles qu'elles sont comprises dans la surface pentagonale de compositions délimitée par les points A, B, C, D et E sur la figure 1.

2. Tamis moléculaire selon la revendication 1, dans lequel les fractions molaires de "M", d'aluminium et de phosphore, présents sous la forme d'oxydes tétraédriques, sont comprises dans la surface hexagonale de compositions délimitée par les points a, b, c, d, e et f sur la figure 2.

3. Tamis moléculaire cristallin selon la revendication 1, dans lequel "M" représente l'arsenic.

4. Tamis moléculaire cristallin selon la revendication 1, dans lequel "M" représente le béryllium.

5. Tamis moléculaire cristallin selon la revendication 1, dans lequel "M" représente le bore.

6. Tamis moléculaire cristallin selon la revendication 1, dans lequel "M" représente le chrome.

7. Tamis moléculaire cristallin selon la revendication 1, dans lequel "M" représente le gallium.

8. Tamis moléculaire cristallin selon la revendication 1, dans lequel "M" représente le lithium.

9. Tamis moléculaire cristallin selon la revendication 1, dans lequel "M" représente le vanadium.

10. Tamis moléculaire cristallin selon la revendication 1 ou 2, ayant un diagramme de diffraction caractéristique de rayons X sous forme de poudre, contenant au moins les intervalles d mentionnés dans le tableau A.

11. Tamis moléculaire cristallin selon la revendication 1 ou 2, ayant un diagramme de diffraction caractéristique de rayons X sous forme de poudre, contenant au moins les intervalles d mentionnés dans le tableau B.

12. Tamis moléculaire cristallin selon la revendication 1 ou 2, ayant un diagramme de diffraction caractéristique de rayons X sous forme de poudre, contenant au moins les intervalles d mentionnés dans le tableau C.

13. Tamis moléculaire cristallin selon la revendication 1 ou 2, ayant un diagramme de diffraction caractéristique de rayons X sous forme de poudre, contenant au moins les intervalles d mentionnés dans le tableau D.

14. Tamis moléculaire cristallin selon la revendication 1 ou 2, ayant un diagramme de diffraction caractéristique de rayons X sous forme de poudre, contenant au moins les intervalles d mentionnés dans le tableau E.

15. Tamis moléculaire cristallin selon la revendication 1 ou 2, ayant un diagramme de diffraction caractéristique de rayons X sous forme de poudre, contenant au moins les intervalles d mentionnés dans le tableau F.

16. Tamis moléculaire cristallin selon la revendication 1 ou 2, ayant un diagramme de diffraction caractéristique de rayons X sous forme de poudre, contenant au moins les intervalles d mentionnés dans le tableau G.

17. Tamis moléculaire cristallin selon la revendication 1 ou 2, ayant un diagramme de diffraction caractéristique de rayons X sous forme de poudre, contenant au moins les intervalles d mentionnés dans le tableau H.

18. Tamis moléculaire cristallin selon la revendication 1 ou 2, ayant un diagramme de diffraction caractéristique de rayons X sous forme de poudre, contenant au moins les intervalles d mentionnés dans le tableau J.

19. Tamis moléculaire cristallin selon la revendication 1 ou 2, ayant un diagramme de diffraction caractéristique de rayons X sous forme de poudre, contenant au moins les intervalles d mentionnés dans le tableau K.

20. Tamis moléculaire cristallin selon la revendication 1 ou 2, ayant un diagramme de diffraction caractéristique de rayons X sous forme de poudre, contenant au moins les intervalles d mentionnés dans le tableau L.

21. Tamis moléculaire cristallin selon la revendication 1 ou 2, ayant un diagramme de diffraction caractéristique de rayons X sous forme de poudre, contenant au moins les intervalles d mentionnés dans le tableau M.

22. Tamis moléculaire cristallin selon la revendication 1 ou 2, ayant un diagramme de diffraction caractéristique de rayons X sous forme de poudre, contenant au moins les intervalles d mentionnés dans le tableau N.

23. Tamis moléculaire cristallin selon la revendication 1 ou 2, ayant un diagramme de diffraction caractéristique de rayons X sous forme de poudre, contenant au moins les intervalles d mentionnés dans le tableau O.

24. Tamis moléculaire cristallin selon la revendication 1 ou 2, ayant un diagramme de diffraction caractéristique de rayons X sous forme de poudre, contenant au moins les intervalles d mentionnés dans le tableau P.

25. Tamis moléculaire cristallin selon la revendication 1 ou 2, ayant un diagramme de diffraction caractéristique de rayons X sous forme de poudre, contenant au moins les intervalles d mentionnés dans le tableau Q.

26. Tamis moléculaire cristallin selon la revendication 1 ou 2, ayant un diagramme de diffraction caractéristique de rayons X sous forme de poudre, contenant au moins les intervalles d mentionnés dans le tableau R.

27. Tamis moléculaire cristallin selon la revendication 1 ou 2, ayant un diagramme de diffraction caractéristique de rayons X sous forme de poudre, contenant au moins les intervalles d mentionnés dans le tableau S.

28. Tamis moléculaire cristallin selon la revendication 1 ou 2, ayant un diagramme de diffraction caractéristique de rayons X sous forme de poudre, contenant au moins les intervalles d mentionnés dans le tableau T.

29. Tamis moléculaire cristallin selon la revendication 1 ou 2, ayant un diagramme de diffraction caractéristique de rayons X sous forme de poudre, contenant au moins les intervalles d mentionnés dans le tableau U.

30. Tamis moléculaire cristallin selon la revendication 1 ou 2, ayant un diagramme de diffraction caractéristique de rayons X sous forme de poudre, contenant au moins les intervalles d mentionnés dans le tableau V.

31. Tamis moléculaire cristallin selon la revendication 1 ou 2, pour l'élaboration duquel on sélectionne au moins deux éléments "M".

32. Tamis moléculaire selon la revendication 31, dans lequel "M" représente l'arsenic et le germanium.

**33.** Tamis moléculaire selon la revendication 31, dans lequel "M" représente le béryllium et le germanium.

**34.** Tamis moléculaire selon la revendication 31, dans lequel "M" représente le chrome et le gallium.

**35.** Tamis moléculaire selon la revendication 31, dans lequel "M" représente l'arsenic et le béryllium.

**36.** Tamis moléculaire selon la revendication 31, dans lequel "M" représente l'arsenic, le vanadium et le béryllium.

**37.** Tamis moléculaire selon la revendication 31, dans lequel "M" représente le germanium et le bore.

**38.** Tamis moléculaire selon la revendication 31, dans lequel "M" représente au moins deux éléments choisis parmi le bore, le chrome, le vanadium, l'arsenic, le germanium et le gallium.

**39.** Tamis moléculaire selon la revendication 31, dans lequel "M" représente le germanium et au moins un élément choisi parmi le béryllium, le bore et le gallium.

**40.** Procédé de préparation du tamis moléculaire cristallin de la revendication 1, selon lequel on forme un mélange réactionnel correspondant à une composition exprimée par la formule suivante en termes de rapports molaires d'oxyde :

$$aR : (M_xAl_yP_z)O_2 : bH_2O$$

dans laquelle "R" représente un agent organique formant matrice ; "a" représente la quantité de "R", et elle peut être égale à zéro ou à une quantité efficace supérieure à zéro ; b a une valeur allant de 0 à environ 500 ; "M" représente au moins deux éléments capables de former des oxydes tétraédriques en réseau et choisis parmi l'arsenic, le béryllium, le bore, le chrome, le gallium, le germanium, le lithium et le vanadium ; "x", "y" et "z" représentent respectivement les fractions molaires de "M", d'aluminium et de phosphore dans le constituant de formule $(M_xAl_yP_z)O_2$, chacun ayant une valeur d'au moins 0,01, de sorte que l'on prépare le tamis moléculaire de la revendication 1.

**41.** Procédé selon la revendication 40, dans lequel "x", "y" et "z" sont compris dans la surface hexagonale de compositions, délimitée par les points F, G, H, I, J et K sur la figure 3 des dessins.

**42.** Procédé selon la revendication 40, dans lequel "a" représente une quantité efficace supérieure à zéro jusqu'à environ 6.

**43.** Procédé selon la revendication 40, dans lequel la source de phosphore dans le mélange réactionnel, est l'acide orthophosphorique.

**44.** Procédé selon la revendication 40, dans lequel la source de phosphore dans le mélange réactionnel, est l'acide orthophosphorique ; et dans lequel la source d'aluminium comprend au moins un composé choisi parmi la pseudo-boéhmite et un alcoolate d'aluminium.

**45.** Procédé selon la revendication 44, dans lequel l'alcoolate d'aluminium est l'isopropylate d'aluminium.

**46.** Procédé selon la revendication 40, dans lequel la source de "M" est choisie parmi les chlorures, les bromures, les iodures, les sulfates, les nitrates, les acétates et les formiates.

**47.** Procédé selon la revendication 40, dans lequel l'agent organique formant matrice est un ammonium quaternaire ou un phosphonium quaternaire de formule :

$$R_4X^+$$

dans laquelle X représente un atome d'azote ou de phosphore et chaque groupe R représente un groupe alkyle ou aryle contenant de 1 à 8 atomes de carbone.

**48.** Procédé selon la revendication 40, dans lequel l'agent organique formant matrice est une amine.

**49.** Procédé selon la revendication 40, dans lequel l'agent formant matrice est choisi parmi l'ion tétrapropyl-lammonium ; l'ion tétraéthylammonium ; la tripropylamine ; la triéthylamine ; la triéthanolamine ; la pipéridine ; la cyclohexylamine ; la 2-méthyl pyridine ; la N,N-diméthylbenzylamine ; la N,N-diéthyléthanolamine ; la choline ; la N,N-diméthylpipérazine ; la 1,4-diaziabicyclo(2,2,2) octane ; la N-méthyldiéthanolamine ; la N-méthyléthanolamine ; la N-méthylpipéridine ; la 3-méthylpipéridine ; la N-méthylcyclohexylamine ; la 3-méthylpyridine ; la 4-méthylpyridine ; la quinuclidine ; l'ion N,N'-diméthyl-1,4-diaziabicyclo (2,2,2) octane ; l'ion tétraméthylammonium ; l'ion tétrabutylammonium ; l'ion tétrapentyllammonium ; la di-n-butylamine ; la néopentylamine ; la di-n-pentylamine ; l'isopropylamine ; la t-butylamine ; l'éthylènediamine ; la pyrrolidine ; la 2-imidazolidone ; la di-n-propylamine et un sel d'ammonium quaternaire polymère de formule $[(C_{14}H_{32}N_2)(OH)_2]_x$ dans laquelle x est égal à au moins 2.

**50.** Tamis moléculaire préparé par calcination des compositions selon la revendication 1 ou la revendication 2, à une température suffisamment élevée pour éliminer au moins une partie de l'agent organique formant matrice présent dans le système de pores intracristallins.

**51.** Procédé de séparation d'une espèce moléculaire mélangée avec des espèces moléculaires ayant un plus faible degré de polarité, selon lequel on met en contact ce mélange d'espèces moléculaires, avec un tamis moléculaire selon la revendication 1, ayant un diamètre de pore suffisamment grand pour adsorber au moins l'une des espèces moléculaires les plus polaires, ce tamis moléculaire étant au moins partiellement activé, de sorte que les molécules de l'espèce moléculaire la plus polaire, sont sélectivement adsorbées dans son système de pores intracristallins.

**52.** Procédé de séparation d'un mélange d'espèces moléculaires ayant différents diamètres cinétiques, selon lequel on met en contact ce mélange avec un tamis moléculaire de la revendication 1 ayant des diamètres de pores suffisamment élevés pour adsorber au moins une, mais pas la totalité des espèces moléculaires de ce mélange, ledit tamis moléculaire étant au moins partiellement activé, de sorte qu'au moins certaines molécules dont les diamètres cinétiques sont suffisamment petits, peuvent pénétrer dans son système de pores intracristallins.

**53.** Procédé selon la revendication 51, dans lequel l'espèce moléculaire la plus polaire est l'eau.

**54.** Procédé de conversion d'un hydrocarbure, selon lequel on met en contact cet hydrocarbure dans des conditions de conversion d'hydrocarbure, avec un tamis moléculaire de la revendication 1 ou de la revendication 2.

**55.** Procédé selon la revendication 54, dans lequel l'opération de conversion d'hydrocarbure, est un craquage.

**56.** Procédé selon la revendication 54, dans lequel l'opération de conversion d'hydrocarbure, est un hydrocraquage.

**57.** Procédé selon la revendication 54, dans lequel l'opération de conversion d'hydrocarbure, est une hydrogénation.

**58.** Procédé selon la revendication 54, dans lequel l'opération de conversion d'hydrocarbure, est une polymérisation.

**59.** Procédé selon la revendication 54, dans lequel l'opération de conversion d'hydrocarbure, est une alkylation.

**60.** Procédé selon la revendication 54, dans lequel l'opération de conversion d'hydrocarbure, est un reformage.

**61.** Procédé selon la revendication 54, dans lequel l'opération de conversion d'hydrocarbure, est un hydrotraitement

**62.** Procédé selon la revendication 54, dans lequel l'opération de conversion d'hydrocarbure, est une isomérisation.

**63.** Procédé selon la revendication 62, dans lequel l'opération de conversion par isomérisation, est une isomérisation du xylène.

**64.** Procédé selon la revendication 54, dans lequel l'opération de conversion d'hydrocarbure, est une déshydrocyclisation.

**Patentansprüche**

**1.** Kristalline Molekularsiebe mit einer dreidimensionalen mikroporösen Gerüststruktur aus $MO_2$, $AlO_2$ und $PO_2$ Tetraeder-Einheiten mit einer empirischen chemischen Zusammensetzung auf wasserfreier Basis, ausgedrückt durch die Formel:

$$mR : (M_xAl_yP_z)O_2$$

in der "R" Wenigstens ein organisches Templatagens darstellt, das in dem intrakristallinen Porensystem vorhanden ist, "m" die molare Menge von "R" pro Mol $(M_xAl_yP_z)O_2$ bedeutet und einen Wert von zwischen 0 und etwa 0,3 hat; und "M" wenigstens ein Element, ausgewählt aus der Gruppe bestehend aus Arsen, Beryllium, Bor, Chrom, Gallium, Germanium, Lithium und Vanadium bedeutet; und "x", "y" und "z" die jeweiligen Molfraktionen von "M", Aluminium und Phosphor bedeuten, die als tetraedrische Oxide vorliegen, wobei diese Molfraktionen dergestalt sind, daß sie innerhalb des pentagonalen Zusammensetzungsgebiets liegen, das durch die Punkte A, B, C, D und E aus der Figur 1 definiert ist.

**2.** Molekularsiebe nach Anspruch 1, bei denen die Molfraktionen von "M", Aluminium und Phosphor als Tetraeder-Oxide vorhanden sind und innerhalb des hexagonalen Zusammensetzungsgebiets liegen, das durch die Punkte a, b, c, d, e und f aus der Figur 2 definiert ist.

**3.** Kristalline Molekularsiebe nach Anspruch 1, worin "M" Arsen ist.

**4.** Kristalline Molekularsiebe nach Anspruch 1, worin "M" Beryllium ist.

**5.** Kristalline Molekularsiebe nach Anspruch 1, worin "M" Bor ist.

**6.** Kristalline Molekularsiebe nach Anspruch 1, worin "M" Chrom ist.

**7.** Kristalline Molekularsiebe nach Anspruch 1, worin "M" Gallium ist.

**8.** Kristalline Molekularsiebe nach Anspruch 1, worin "M" Lithium ist.

**9.** Kristalline Molekularsiebe nach Anspruch 1, worin "M" Vanadium ist.

**10.** Kristalline Molekularsiebe nach Anspruch 1 oder 2 mit einem charakteristischen Röntgenbeugungsmuster nach der Kristallpulvermethode, das wenigstens die d-Werte aus Tabelle A enthält.

**11.** Kristalline Molekularsiebe nach Anspruch 1 oder 2 mit einem charakteristischen Röntgenbeugungsmuster nach der Kristallpulvermethode, das wenigstens die d-Werte aus Tabelle B enthält.

**12.** Kristalline Molekularsiebe nach Anspruch 1 oder 2 mit einem charakteristischen Röntgenbeugungsmuster nach der Kristallpulvermethode, das wenigstens die d-Werte aus Tabelle C enthält.

**13.** Kristalline Molekularsiebe nach Anspruch 1 oder 2 mit einem charakteristischen Röntgenbeugungsmuster nach der Kristallpulvermethode, das wenigstens die d-Werte aus Tabelle D enthält.

**14.** Kristalline Molekularsiebe nach Anspruch 1 oder 2 mit einem charakteristischen Röntgenbeugungsmuster nach der Kristallpulvermethode, das wenigstens die d-Werte aus Tabelle E enthält.

**15.** Kristalline Molekularsiebe nach Anspruch 1 oder 2 mit einem charakteristischen Röntgenbeugungsmuster nach der Kristallpulvermethode, das wenigstens die d-Werte aus Tabelle F enthält.

16. Kristalline Molekularsiebe nach Anspruch 1 oder 2 mit einem charakteristischen Röntgenbeugungsmuster nach der Kristallpulvermethode, das wenigstens die d-Werte aus Tabelle G enthält.

17. Kristalline Molekularsiebe nach Anspruch 1 oder 2 mit einem charakteristischen Röntgenbeugungsmuster nach der Kristallpulvermethode, das wenigstens die d-Werte aus Tabelle H enthält.

18. Kristalline Molekularsiebe nach Anspruch 1 oder 2 mit einem charakteristischen Röntgenbeugungsmuster nach der Kristallpulvermethode, das wenigstens die d-Werte aus Tabelle J enthält.

19. Kristalline Molekularsiebe nach Anspruch 1 oder 2 mit einem charakteristischen Röntgenbeugungsmuster nach der Kristallpulvermethode, das wenigstens die d-Werte aus Tabelle K enthält.

20. Kristalline Molekularsiebe nach Anspruch 1 oder 2 mit einem charakteristischen Röntgenbeugungsmuster nach der Kristallpulvermethode, das wenigstens die d-Werte aus Tabelle L enthält.

21. Kristalline Molekularsiebe nach Anspruch 1 oder 2 mit einem charakteristischen Röntgenbeugungsmuster nach der Kristallpulvermethode, das wenigstens die d-Werte aus Tabelle M enthält.

22. Kristalline Molekularsiebe nach Anspruch 1 oder 2 mit einem charakteristischen Röntgenbeugungsmuster nach der Kristallpulvermethode, das wenigstens die d-Werte aus Tabelle N enthält.

23. Kristalline Molekularsiebe nach Anspruch 1 oder 2 mit einem charakteristischen Röntgenbeugungsmuster nach der Kristallpulvermethode, das wenigstens die d-Werte aus Tabelle O enthält.

24. Kristalline Molekularsiebe nach Anspruch 1 oder 2 mit einem charakteristischen Röntgenbeugungsmuster nach der Kristallpulvermethode, das wenigstens die d-Werte aus Tabelle P enthält.

25. Kristalline Molekularsiebe nach Anspruch 1 oder 2 mit einem charakteristischen Röntgenbeugungsmuster nach der Kristallpulvermethode, das wenigstens die d-Werte aus Tabelle Q enthält.

26. Kristalline Molekularsiebe nach Anspruch 1 oder 2 mit einem charakteristischen Röntgenbeugungsmuster nach der Kristallpulvermethode, das wenigstens die d-Werte aus Tabelle R enthält.

27. Kristalline Molekularsiebe nach Anspruch 1 oder 2 mit einem charakteristischen Röntgenbeugungsmuster nach der Kristallpulvermethode, das wenigstens die d-Werte aus Tabelle S enthält.

28. Kristalline Molekularsiebe nach Anspruch 1 oder 2 mit einem charakteristischen Röntgenbeugungsmuster nach der Kristallpulvermethode, das wenigstens die d-Werte aus Tabelle T enthält.

29. Kristalline Molekularsiebe nach Anspruch 1 oder 2 mit einem charakteristischen Röntgenbeugungsmuster nach der Kristallpulvermethode, das wenigstens die d-Werte aus Tabelle U enthält.

30. Kristalline Molekularsiebe nach Anspruch 1 oder 2 mit einem charakteristischen Röntgenbeugungsmuster nach der Kristallpulvermethode, das wenigstens die d-Werte aus Tabelle V enthält.

31. Kristalline Molekularsiebe nach Anspruch 1 oder 2, worin wenigstens zwei Elemente "M" ausgewählt sind.

32. Molekularsiebe nach Anspruch 31, worin "M" Arsen und Germanium ist.

33. Molekularsiebe nach Anspruch 31, worin "M" Beryllium und Germanium ist.

34. Molekularsiebe nach Anspruch 31, worin "M" Chrom und Gallium ist.

35. Molekularsiebe nach Anspruch 31, worin "M" Arsen und Beryllium ist.

**36.** Molekularsiebe nach Anspruch 31, worin "M" Arsen, Vanadium und Beryllium ist.

**37.** Molekularsiebe nach Anspruch 31, worin "M" Germanium und Bor ist.

**38.** Molekularsiebe nach Anspruch 31, worin "M" aus wenigstens zwei Elementen, ausgewählt aus der aus Bor, Chrom, Vanadium, Arsen, Germanium und Gallium bestehenden Gruppe ausgewählt ist.

**39.** Molekularsiebe nach Anspruch 31, worin "M" Germanium und wenigstens ein Element, ausgewählt aus der Gruppe bestehend aus Beryllium, Bor und Gallium, ist.

**40.** Verfahren zur Herstellung von kristallinen Molekularsieben nach Anspruch 1, daß das Bereitstellen einer Reaktionsmischung mit einer Zusammensetzung zahlenmäßig ausgedrückt durch die Molverhältnisse der Oxide wie folgt umfaßt:

$$aR : (M_xAl_yP_z)O_2 : bH_2O$$

in der "R" ein organisches Templatagens ist: "a" die Menge von "R" darstellt und Null sein kann oder eine wirksame Menge größer als Null: "b" einen Wert von zwischen Null bis etwa 500 hat, "M" wenigstens zwei Elemente bedeutet, die eine Gerüststruktur aus Tetraeder-Oxiden bilden können und ausgewählt sind aus der aus Arsen, Beryllium, Bor, Chrom, Gallium, Germanium, Lithium und Vanadium bestehenden Gruppe; "x", "y" und "z" bedeuten die jeweiligen Molfraktionen von "M", Aluminium und Phosphor in dem $(M_xAl_yP_z)O_2$ Bestandteil, wobei jeder einen Wert von wenigstens 0,01 hat und die Molekularsiebe gemäß Anspruch 1 hergestellt werden.

**41.** Verfahren nach Anspruch 40, worin "x", "y" und "z" innerhalb eines hexagonalen Zusammensetzungsgebietes liegen, das durch die Punkte F, G, H, I, J, und K definiert ist, was Figur 3 der Zeichnungen darstellt.

**42.** Verfahren nach Anspruch 40, worin "a" eine wirksame Menge von größer als Null bis etwa 6 ist.

**43.** Verfahren nach Anspruch 40, worin die Quelle für Phosphor in der Reaktionsmischung Orthophosphorsäure ist.

**44.** Verfahren nach Anspruch 40, worin die Quelle für Phosphor in der Reaktionsmischung Orthophosphorsäure ist und die Quelle für Aluminium wenigstens eine Verbindung ausgewählt aus der Gruppe Pseudoboehmit und Aluminiumalkoxid ist.

**45.** Verfahren nach Anspruch 44, worin das Aluminiumalkoxid Aluminiumisopropoxid ist.

**46.** Verfahren nach Anspruch 40, worin die Quelle für "M" ausgewählt wird aus der Gruppe bestehend aus den Chloriden, Bromiden, Jodiden, Sulfaten, Nitraten, Acetaten und Formiaten.

**47.** Verfahren nach Anspruch 40, worin das organische Templatagens eine quaternäre Ammonium- oder quaternäre Phosphoniumverbindung ist mit der Formel:

$$R_4X^+$$

in der X Stickstoff oder Phosphor und R jeweils eine Alkyl- ode Arylgruppe mit 1 bis 8 Kohlenstoffatomen ist.

**48.** Verfahren nach Anspruch 40, worin das organische Templatagens ein Amin ist.

**49.** Verfahren nach Anspruch 40, worin das Templatagens ausgewählt ist aus der Gruppe bestehend aus Tetrapropylammoniumion, Tetraethylammoniumion, Tripropylamin, Triethylamin, Triethanolamin, Piperidin, Cyclohexylamin, 2-Methylpyridin, N,N-Dimethylbenzylamin, N,N-Diethylethanolamin, Cholin, N,N-Dimethylpiperazin, 1,4-Diazabicyclo(2,2,2)octan, N-Methyldiethanolamin, N-Methylethanolamin, N-Methylpiperidin, 3-Methylpiperidin, N-Methylcyclohexylamin, 3-Methylpyridin, 4-Methylpyridin, Chinuclidin, N,N'-Dimethyl-1,4-diazabicyclo(2,2,2)octanion, Tetramethylammoniumion, Tetrabutylammoniumion, Tetrapentylammoniumion, Di-n-butylamin, Neopentylamin, Di-n-pentylamin, Isopropylamin, t-Butylamin, Ethylendiamin, Pyrrolidin, 2-Imidazolidon, Di-n-propylamin und einem polymeren quaternären Ammoniumsalz $[(C_{14}H_{32}N_2)(OH)_2]_x$, worin x einen Wert von wenigstens 2 hat.

50. Molekularsieb hergestellt durch Calcinieren der Zusammensetzungen gemäß Anspruch 1 oder Anspruch 2, bei einer Temperatur, die ausreichend hoch ist, um wenigstens einen Teil des organischen Templat-Agens, das in dem intrakristallinen Porensystem anwesend ist, zu entfernen.

51. Verfahren zur Trennung von molekularen Sorten aus einem Gemisch mit molekularen Sorten, die einen geringeren Polaritätsgrad aufweisen, umfassend das in Kontakt bringen dieser Mischung molekularer Sorten mit einem Molekularsieb nach Anspruch 1 mit einem Porendurchmesser, der groß genug ist, wenigstens eine der polareren molekularen Sorte zu adsorbieren, wobei dieses Molekularsieb wenigstens teilweise aktiviert ist und Moleküle der polareren molekularen Sorte selektiv an das intrakristalline Porensystem adsorbiert werden.

52. Verfahren zur Trennung eines Gemisches von molekularen Sorten mit unterschiedlichen kinetischen Durchmessern, umfassend das in Kontakt bringen dieses Gemischs mit einem Molekularsieb gemäß Anspruch 1 mit einem Porendurchmesser der groß genug ist wenigstens eine jedoch nicht alle molekularen Sorten dieses Gemischs zu adsorbieren, wobei dieses Molekularsieb wenigstens teilweise aktiviert wird und wenigstens einige Moleküle, deren kinetische Durchmesser ausreichend klein sind, in das intrakristalline Porensystem eindringen können.

53. Verfahren nach Anspruch 51, in dem die polarere molekulare Sorte Wasser ist.

54. Verfahren zur Umwandlung eines Kohlenwasserstoffs, umfassend das in Kontakt bringen dieses Kohlenwasserstoffs unter Kohlenwasserstoff-Umwandlungs-Bedingungen mit einem Molekularsieb gemäß Anspruch 1 oder Anspruch 2.

55. Verfahren nach Anspruch 54, in dem der Kohlenwasserstoff-Umwandlungsprozeß Cracken ist.

56. Verfahren nach Anspruch 54, in dem der Kohlenwasserstoff-Umwandlungsprozeß Hydrocracken ist.

57. Verfahren nach Anspruch 54, in dem der Kohlenwasserstoff-Umwandlungsprozeß eine Hydrierung ist.

58. Verfahren nach Anspruch 54, in dem der Kohlenwasserstoff-Umwandlungsprozeß eine Polymerisation ist.

59. Verfahren nach Anspruch 54, in dem der Kohlenwasserstoff-Umwandlungsprozeß eine Alkylierung ist.

60. Verfahren nach Anspruch 54, in dem der Kohlenwasserstoff-Umwandlungsprozeß eine Reformierung ist.

61. Verfahren nach Anspruch 54, in dem der Kohlenwasserstoff-Umwandlungsprozeß ein "Hydrotreating" ist.

62. Verfahren nach Anspruch 54, in dem der Kohlenwasserstoff-Umwandlungsprozeß eine Isomerisierung ist.

63. Verfahren nach Anspruch 62, in dem der Isomerisierungs-Umwandlungsprozeß eine Xylenisomerisierung ist.

64. Verfahren nach Anspruch 54, in dem der Kohlenwasserstoff-Umwandlungsprozeß eine Dehydrocyclisierung ist.

F I G.  1

FIG. 2

F I G. 3

EP 0 158 976 B1